# EUROPEAN PATENT APPLICATION

(11) **EP 4 137 574 A1**
(43) Date of publication of application: **22.02.2023**
(21) Application number: 21788761.1
(22) Date of filing: 16.04.2021
(51) Int. Cl.: C12N 15/13, A61K 9/08, A61K 39/395, A61P 35/00, A61P 35/02, A61P 37/06, C07K 1/18, C07K 16/28, C07K 16/46

(54) **COLOR REMOVAL METHOD FOR PROTEIN FORMULATION ACTIVE INGREDIENT**

(30) Priority: 17.04.2020 JP 2020073753
(71) Applicant: ONO Pharmaceutical Co., Ltd., Osaka-shi, Osaka 541-8526 (JP)
(72) Inventor: NOZAKI, Shinya, Mishima-gun, Osaka 618-8585 (JP); ARIMA, Naoki, Mishima-gun, Osaka 618-8585 (JP)
(74) Representative: Grünecker Patent- und Rechtsanwälte PartG mbB
(86) International application number: PCT/JP2021/015665
(87) International publication number: WO 2021/210662

(57) **Abstract**

A problem of the present invention is to provide a method for removing coloration from a drug substance solution of protein preparation, in particular, antibody preparation, a method for preparing drug substance solution of protein preparations including it, as a part thereof, and highly concentrated, colorless drug substance solutions thereof.

The present invention provides a method for removing terminal glycation products causing the coloration in the drug substance solution of protein preparation, in particular, antibody preparation, by anion-exchange chromatography. This method has made it possible to provide colorless drug substance solutions.

## Description

### [Technical Field]

The present invention relates to methods for removing color from a drug substance solution of protein preparation and solutions produced by manufacturing processes including the same method.

### [Background Art]

A form of subcutaneous injection is often chosen as those of prescription for antibody drugs because of its convenience and patient burden. In the case of subcutaneous injection, a higher concentration of the drug is naturally required due to the limitation of dose thereof, in particular, in the case of antibody drugs, which includes several problems due to that. One of those problems is the coloration of drug substance solution, and the European Pharmacopoeia requires that the color of drug substance solution be colorless or slightly colored. In addition, the coloration of drug substance solution may indicate the presence of impurities and the associated heterogeneity thereof, and the probability that it degrades the performance of drug, or affects its efficacy or safety cannot be denied (Non-Patent Literatures 1 and 2).

One of causes of such coloration is a culture environment for producing cells. It has been reported that since particularly, the culture of CHO cells, which are widely used for antibody production, is carried out under high oxygen and high glucose conditions, proteins produced are exposed to marked oxidation and glycation environment, by which forms Advanced Glycation End products (AGEs), and a part of which is a cause of coloration (Non-Patent Literatures 3, 4 and 5). Furthermore, on the other hand, it has been reported that such advanced glycation end products may be responsible for the reduced efficacy of protein drugs, and the removal of causative substance from colored drug substance solution is important to maintain the quality and performance of drug.

### [Citation List]

### [Patent literature]

Non-Patent Literature 1: Anal. Chem. 2014, 86, 9816-9823
Non-Patent Literature 2: Biotechnology Progress (2013), 29 (5), 1270-1277
Non-Patent Literature 3: European Pharmacopoeia, 8.2 ed.; Council of Europe: Strasbourg, France, 2014.
Non-Patent Literature 4: Analytical Chemistry (2013), 85 (23), 11401-11409
Non-Patent Literature 5: BMC Ophthalmol. 2006, 6, 10

### [Summary of Invention]

### [Technical Problem]

The purpose of the present invention is to provide methods for removing color from drug substance solution of protein preparation and solutions produced by manufacturing processes including the same method.

### [Solution to Problem]

The present inventors diligently studied in order to find methods, and have found methods for removing substances causing the coloration of drug substance solution by ion-exchange chromatography, and have completed the present invention.

That is, the present invention relates to the followings.
[1] A method for separating non-colored proteins from a solution containing non-colored proteins and colored proteins, comprising
   (i) a step for loading the solution onto an ion-exchange carrier column, and using an equilibration buffer at a predetermined initial pH, making the non-colored proteins and colored proteins bind to ion-exchange carriers thereof,
   (ii) a step for flowing an elution buffer, forming a pH gradient varying from the initial pH to a terminal pH, through the ion-exchange carrier column at a predetermined flow rate, under a condition predetermined to separate the non-colored proteins and colored proteins, and
   (iii) a step for collecting a predetermined fraction containing the non-colored proteins eluted by the preceding step (ii);
[2] a method for separating non-colored proteins from a solution containing non-colored proteins and colored proteins, comprising
   (i) a step for loading the solution onto an ion-exchange carrier column,
   (ii) a step for flowing an equilibration buffer prepared so that the colored proteins in the solution bind to ion-exchange carriers thereof and the non-colored proteins do not, through the ion-exchange carrier column at a predetermined flow rate, and
   (iii) a step for collecting a fraction containing the non-colored proteins flowed through in the preceding step (ii) as it is;
[3] the separation method according to the preceding item [1] or [2], wherein the color concentration of the solution containing non-colored proteins and colored proteins or concentrated solution thereof is (1) in the visual comparison test with the reference standard solution BY5 of item 2.65 "color comparison test" in the general tests of the Japanese Pharmacopoeia, 17th Edition, more than that of BY5, or (2) the value calculated by multiplying the ratio of the peak area of fluorescence intensity (provided that the excitation wavelength is 380 nm and the detection wavelength is 560 nm, which may be abbreviated as "Em560(ex380)" hereinafter) to the peak area of UV absorbance at a wavelength of 280 nm, calculated based on the UV absorption measurement and fluorescence spectrum measurement of the solution or concentrated solution thereof (hereinafter, may being abbreviated as "UV₂₈₀"), by 100 (hereinafter, may being abbreviated as "Em/UV*100 value") is higher than about 6.3;
[4] the separation method according to any one of the preceding items [1] to [3], wherein the ion-exchange carrier is an anion-exchange carrier;
[5] the separation method according to any one of the preceding items [1] to [4], wherein the ion-exchange carrier or anion-exchange carrier is in the form of small particles, membranes or monoliths;
[6] the separation method according to the preceding item [4] or [5], wherein the anion-exchange carrier is a strong anion-exchange carrier, weak anion-exchange carrier or multi-mode anion-exchange carrier;
[7] the separation method according to the preceding item [4] or [5], wherein the anion-exchange carrier is a weak anion-exchange carrier or multi-mode anion-exchange carrier;
[8] the separation method according to the preceding item [7], wherein the weak anion-exchange carrier is any one selected from the group consisting of Fractogel (registered trademark) EMD DEAE, Fractogel (registered trademark) EMD DMAE, Capto (registered trademark) DEAE, DEAE Ceramic HyperD (registered trademark) F, Toyopearl (registered trademark) NH2-750F, TOYOPEARL (registered trademark) DEAE-650C, TOYOPEARL (registered trademark) DEAE-650M, TOYOPEARL (registered trademark) DEAE-650S, Cellufine (registered trademark) A-200, Cellufine (registered trademark) A-500, Cellufine (registered trademark) A-800 and Cellufine (registered trademark) MAX DEAE;
[9] The separation method according to the preceding item [7], wherein the multi-mode anion-exchange carrier is any one selected from the group consisting of Toyopearl (registered trademark) NH2-750F, Capto (registered trademark) Adhere, Capto (registered trademark) Adhere ImpRes, Capto (registered trademark) MMC, Capto (registered trademark) core 700 and Cellufine (registered trademark) IB (preferably, Toyopearl (registered trademark) NH2-750F);
[10] the separation method according to the preceding item [6], wherein the strong anion-exchange carrier is any one selected from the group consisting of Fractogel (registered trademark) EMD TMAE (M), Fractogel (registered trademark) EMD TMAE Medcap (M), Fractogel (registered trademark) EMD TMAE Hicap (M), Eshmuno (registered trademark) Q, Eshmuno (registered trademark) QPX, Eshmuno (registered trademark) QPX Hicap, Capto (registered trademark) Q, Capto (registered trademark) Q ImpRes, Q Sepharose (registered trademark) FF, Q Sepharose (registered trademark) HP, Q Sepharose (registered trademark) XL, Source (registered trademark) 30Q, Capto (registered trademark) Adhere, Capto (registered trademark) Adhere ImpRes, POROS (registered trademark) 50 HQ, POROS (registered trademark) 50 XQ, POROS (registered trademark) 50 PI, Q HyperCel (registered trademark), Toyopearl (registered trademark) GigaCap Q 650M, Toyopearl (registered trademark) GigaCap Q 650S, Toyopearl (registered trademark) Super Q, YMC (registered trademark) BioPro Q, Macro-Prep (registered trademark) High Q, Nuvia (registered trademark) Q, UNOsphere (registered trademark) Q, Cellufine (registered trademark) Q-500, Cellufine (registered trademark) MAX Q-r and Cellufine (registered trademark) MAX Q-h;
[11] The separation method according to any one of the preceding items [1] to [10], wherein the isoelectric point of the colored proteins falls within the range of from about 5.0 to about 9.1 (preferably from about 6.0 to about 7.0);
[12] the separation method according to any one of the preceding items [1] to [11], wherein the isoelectric point of the non-colored proteins falls within any range selected from the group consisting of about 7.0 or more but less than about 7.5, about 7.5 or more but less than about 8.0, about 8.0 or more but less than about 8.5, about 8.5 or more but less than about 9.0, and about 9.0 or more but less than about 9.5;
[13] the separation method according to any one of the preceding items [1] to [12], wherein the total concentration of the non-colored proteins and the colored proteins which are loaded into the ion-exchange carrier column is from about 2 to about 50 mg/mL (preferably from about 10 to about 30 mg/mL);
[14] the separation method according to the preceding item [13], wherein the total concentration is any concentration selected from the group consisting of about 5 mg/mL, about 10 mg/mL, about 15 mg/mL, about 20 mg/mL, about 25 mg/mL, about 30 mg/mL, about 35 mg/mL, about 40 mg/mL, about 45 mg/mL and about 50 mg/mL;
[15] the separation method according to any one of the preceding items [1] to [12], wherein the total loading amount of the non-colored proteins and colored proteins which are loaded into the ion-exchange carrier column is from about 2 to about 200 mg/mL;
[16] the separation method according to the preceding item [15], wherein the total loading amount of the non-colored proteins and the colored proteins which are loaded into the ion-exchange carrier column is any one selected from the group consisting of about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL and about 200 mg/mL;
[17] the separation method according to any one of the preceding items [1] and [3] to [16], wherein the range of pH gradient from the initial pH to the terminal pH is set so that the respective isoelectric points of the colored protein and/or non-colored protein is/are interposed between the same range;
[18] the separation method according to any one of the preceding items [1] and [3] to [17], wherein the pH gradient descends from the initial pH to the terminal pH;
[19] the separation method according to any one of the preceding items [1] and [3] to [18], wherein the initial pH is an arbitrary pH within the range of from about 11.0 to about 7.0 (specifically, any pH selected from the group consisting of about 11.0, about 10.8, about 10.6, about 10.4, about 10.2, about 10.0, about 9.8, about 9.6, about 9.4, about 9.2, about 9.0, about 8.8, about 8.6, about 8.4, about 8.2, about 8.0, about 7.8, about 7.6, about 7.4, about 7.2 and about 7.0);
[20] the separation method according to any one of the preceding items [1] and [3] to [19], wherein the terminal pH is an arbitrary pH within the range of from about 4.0 to about 8.2 (specifically, any pH selected from the group consisting of about 4.0, about 4.2, about 4.4, about 4.6, about 4.8, about 5.0, about 5.2, about 5.4, about 5.6, about 5.8, about 6.0, about 6.2, about 6.4, about 6.6, about 6.8, about 7.0, about 7.2, about 7.4, about 7.6, about 7.8, about 8.0 and about 8.2) (provided that the initial pH is at least 1.0 higher than the terminal pH);
[21] the separation method according to any one of the preceding items [1] and [3] to [18], wherein the range of the pH gradient is that from any one of the initial pH selected from the group consisting of about 11.0, about 10.8, about 10.6, about 10.4, about 10.2, about 10.0, about 9.8, about 9.6, about 9.4, about 9.2, about 9.0, about 8.8, about 8.6, about 8.4, about 8.2, about 8.0, about 7.8, about 7.6, about 7.4, about 7.2 and about 7.0 to any one of the terminal pH selected from the group consisting of about 4.0, about 4.2, about 4.4, about 4.6, about 4.8, about 5.0, about 5.2, about 5.4, about 5.6, about 5.8, about 6.0, about 6.2, about 6.4, about 6.6, about 6.8, about 7.0, about 7.2, about 7.4, about 7.6, about 7.8, about 8.0 and about 8.2 (preferably, the initial pH is at least 2.0 higher than the terminal pH);
[22] the separation method according to any one of the preceding items [1] and [3] to [21], wherein the pH gradient is a linear gradient or step-wise gradient;
[23] the separation method according to any one of the preceding items [1] and [3] to [22], wherein the elution buffer described in the preceding item [1] consists of a combination of the buffer at the initial pH (hereinafter, may being abbreviated as "buffer A") and the buffer at the terminal pH (hereinafter, may being abbreviated as "buffer B") at an arbitrary ratio thereof;
[24] the separation method according to the preceding item [23], wherein the buffer A and/or buffer B consists of a combination of a plurality of good buffers selected from the group consisting of MES, Bis-Tris, ADA, PIPES, ACES, MOPSO, BES, MOPS, TES, HEPES, TAPSO, POPSO, HEPSO, EPPS, Tricine, Bicine, TAPS, CHES, CAPSO and CAPS, respectively;
[25] the separation method according to the preceding item [23], wherein the buffer A and/or buffer B consists of a combination of a plurality of good buffers selected from the group consisting of MES, MOPS, TAPS and CAPSO (preferably a combination of MES, MOPS, TAPS and CAPSO), respectively;
[26] the separation method according to the preceding item [23], wherein the buffer A is Thermo Scientific (registered trademark) CX-1 pH Gradient Buffer B (pH 10.2) and the buffer B is Thermo Scientific (registered trademark) CX-1 pH Gradient Buffer A (pH 5.6);
[27] the separation method according to the preceding item [23], wherein the buffer A and/or buffer B consists of a combination of a plurality of buffers selected from the group consisting of Tris-acetate, Tris-phosphate and Tris-citrate, respectively;
[28] the separation method according to any one of the preceding items [23] to [27], wherein the pH gradient with the elution buffer is performed by continuously or step-by-step changing the ratio of the combination of the buffer A and buffer B;
[29] the separation method according to any one of the preceding items [23] to [28], wherein the pH gradient is the step-wise gradient which is performed by increasing the percentage (%) of the buffer B in the elution buffer by an arbitrary percentage in the range of from about 0.5% to about 5% (specifically, any percentage selected from the group consisting of about 0.5%, about 1%, about 1.5%, about 2%, about 2.5%, about 3%, about 3.5%, about 4%, about 4.5% and about 5%) at an interval corresponding to an arbitrary column volume in the range of from about 1 to about 20 CV (specifically, any column volume selected from the group consisting of about 1 CV, about 2 CV, about 3 CV, about 4 CV, about 5 CV, about 6 CV, about 7 CV, about 8 CV, about 9 CV, about 10 CV, about 15 CV and about 20 CV);
[30] the separation method according to any one of the preceding items [23] to [28], wherein the pH gradient is the step-wise gradient which is performed by decreasing the pH of the elution buffer by an arbitrary value in the range of from about 0.1 to about 1.0 (specifically, any value selected from the group consisting of about 0.1, about 0.2, about 0.3, about 0.4, about 0.5, about 0.6, about 0.7, about 0.8, about 0.9 and about 1.0) at an interval corresponding to an arbitrary column volume in the range of from about 1 to about 20 CV (specifically, any column volume selected from the group consisting of about 1 CV, about 2 CV, about 3 CV, about 4 CV, about 5 CV, about 6 CV, about 7 CV, about 8 CV, about 9 CV, about 10 CV, about 15 CV and about 20 CV);
[31] the separation method according to any one of the preceding items [23] to [28], wherein the pH gradient is the step-wise gradient which is performed by increasing the percentage (%) of the buffer B in the elution buffer by an arbitrary percentage in the range of from about 0.5% to about 5% (specifically, any percentage selected from the group consisting of about 0.5%, about 1%, about 1.5%, about 2%, about 2.5%, about 3%, about 3.5%, about 4%, about 4.5% and about 5%) within a range corresponding to an arbitrary column volume in the range of from about 20 to about 50 CV (specifically, any column volume selected from the group consisting of about 20 CV, about 25 CV, about 30 CV, about 35 CV, about 40 CV, about 45 CV and about 50 CV);
[32] the separation method according to any one of the preceding items [23] to [28], wherein the pH gradient is the step-wise gradient which is performed by decreasing the pH of the elution buffer by an arbitrary value in the range of from about 0.1 to about 1.0 (specifically, any value selected from the group consisting of about 0.1, about 0.2, about 0.3, about 0.4, about 0.5, about 0.6, about 0.7, about 0.8, about 0.9 and about 1.0) within a range corresponding to an arbitrary column volume in the range of from about 20 to about 50 CV (specifically, any column volume selected from the group consisting of about 20 CV, about 25 CV, about 30 CV, about 35 CV, about 40 CV, about 45 CV and about 50 CV);
[33] the separation method according to any one of the preceding items [23] to [28], wherein the pH gradient is the liner gradient which is performed by continuously increasing the percentage (%) of the buffer B in the elution buffer within a range corresponding to an arbitrary column volume in the range of from about 20 to about 50 CV (specifically, any column volume selected from the group consisting of about 20 CV, about 25 CV, about 30 CV, about 35 CV, about 40 CV, about 45 CV and about 50 CV);
[34] the separation method according to any one of the preceding items [23] to [28], wherein the pH gradient is the liner gradient which is performed by continuously decreasing the pH of the elution buffer within a range corresponding to an arbitrary column volume in the range of from about 20 to about 50 CV (specifically, any column volume selected from the group consisting of about 20 CV, about 25 CV, about 30 CV, about 35 CV, about 40 CV, about 45 CV and about 50 CV);
[35] the separation method according to any one of the preceding items [29], [31] and [33], wherein the percentage (%) of the buffer B is increased step-by-step or continuously from an arbitrary percentage in at least the range of from about 10 to about 100% (specifically, any percentage selected from the group consisting of about 10 %, about 20 %, about 30 %, about 40 %, about 50 %, about 60 %, about 70 %, about 80 %, about 90 % and about 100%) to an arbitrary percentage in the range of from about 30 % to about 100 % (preferably, any percentage selected from the group consisting of about 30 %, about 40 %, about 50 %, about 60 %, about 70 %, about 80 %, about 90 % and about 100 %);
[36] the separation method according to any one of the preceding items [23] to [28], wherein the pH gradient is performed (a) by increasing the percentage (%) of the buffer B in the elution buffer step-by-step by an arbitrary percentage in the range of from about 1 to about 2 %, at an interval corresponding to an arbitrary column volume in the range of from about 4 to about 6 CV, over at least the range of from about 50 to 100%, or (b) by decreasing the pH of the elution buffer step-by-step by an arbitrary pH in the range of from about 0.6 to about 1.0, within a range corresponding to the column volume of from about 30 to about 40 CV;
[37] the separation method according to any one of the preceding items [1] to [36], wherein the respective linear flow rates of the elution buffer and the equilibration buffer described in the preceding item [2] are arbitrary linear flow rates in the range of from about 100 to about 800 cm/h (specifically, any linear flow rate selected from the group consisting of about 100 cm/h, about 150 cm/h, about 200 cm/h, about 250 cm/h, about 300 cm/h, about 350 cm/h, about 400 cm/h, about 450 cm/h, about 500 cm/h, about 550 cm/h, about 600 cm/h, about 650 cm/h, about 700 cm/h, about 750 cm/h and about 800 cm/h);
[38] the separation method according to any one of the preceding items [1] and [3] to [37], wherein the electrical conductivity of the equilibration buffer and/or elution buffer described in the preceding item [1] is an arbitrary electrical conductivity in the range of from about 1 to about 20 mS/cm (preferably, any electrical conductivity in the range of from about 2 to about 15 mS/cm, and specifically, any electrical conductivity selected from the group consisting of about 2 mS/cm, about 3 mS/cm, about 4 mS/cm, about 5 mS/cm, about 6 mS/cm, about 7 mS/cm, about 8 mS/cm, about 9 mS/cm, about 10 mS/cm, about 11 mS/cm, about 12 mS/cm, about 13 mS/cm, about 14 mS/cm and about 15 mS/cm.);
[39] the separation method according to any one of the preceding items [2] to [16], wherein the electrical conductivity of the equilibration buffer described in the preceding item [2] is an arbitrary electrical conductivity in the range of from about 1 to about 20 mS/cm (preferably, an arbitrary electrical conductivity in the range of from about 2 to about 15 mS/cm, specifically, any electrical conductivity selected from the group consisting of about 2 mS/cm, about 3 mS/cm, about 4 mS/cm, about 5 mS/cm, about 6 mS/cm, about 7 mS/cm, about 8 mS/cm, about 9 mS/cm, about 10 mS/cm, about 11 mS/cm, about 12 mS/cm, about 13 mS/cm, about 14 mS/cm and about 15 mS/cm, and more preferably an arbitrary electrical conductivity in the range of from about 5 to about 7 mS/cm);
[40] the separation method according to any one of the preceding items [2] to [16] and [39], wherein the pH of the equilibration buffer described in the preceding item [2] is an arbitrary pH in the range of from about 9.2 to about 7.4 (specifically, selected from the group consisting of about 9.2, about 9.0, about 8.8, about 8.6, about 8.4, about 8.2, about 8.0, about 7.8, about 7.6 and about 7.4, and preferably an arbitrary pH in the range of from about 8.2 to about 7.8);
[41] the separation method according to any one of the preceding items [1] and [3] to [38], wherein the electrical conductivities of the equilibration buffer and elution buffer described in the preceding item [1] are substantially the same;
[42] the separation method according to any one of the preceding items [1] to [41], which includes a step for washing the ion-exchange carrier with a washing buffer, as necessary, in one or two or more of steps (i) to (iii) of the separation method described in the preceding item [1], or in one or two or more of steps (i) to (iii) of the separation method described in the preceding item [2];
[43] the separation method according to any one of the preceding items [1] to [42], wherein the color concentration of the collected or recovered fraction of the non-colored proteins or concentrated solution thereof is substantially the same as or lighter than that of BY5 in the visual comparison test with the reference standard solution BY5 in the "color comparison test" of the Japanese Pharmacopoeia;
[44] the separation method according to any one of the preceding items [1] to [42], wherein the color concentration of the collected or recovered fraction of the non-colored proteins or concentrated solution thereof is substantially the same as or lighter than that of BY6 in the visual comparison test with the reference standard solution BY6 in the "color comparison test" of the Japanese Pharmacopoeia;
[45] the separation method according to any one of the preceding items [1] to [42], wherein the color concentration of the collected or recovered fraction of the non-colored proteins or concentrated solution thereof is substantially the same as that of BY7 in the visual comparison test with the reference standard solution BY7 in the "color comparison test" of the Japanese Pharmacopoeia;
[46] the separation method according to any one of the preceding items [1] to [42], wherein the Em/UV*100 value of the collected or recovered fraction of the non-colored proteins or concentrated solution thereof is about 6.3 or less;
[47] the separation method according to any one of the preceding items [1] to [42], wherein the Em/UV*100 value of the collected or recovered fraction of the non-colored proteins or concentrated solution thereof is about 4.1 or less;
[48] the separation method according to any one of the preceding items [3] and [43] to [47], wherein the protein concentration of the concentrated solution is at least an arbitrary concentration in the range of from about 50 to about 100 mg/mL (specifically, at least any concentration selected from the group consisting of about 50 mg/mL, about 60 mg/mL, about 70 mg/mL, about 80 mg/mL, about 90 mg/mL and about 100 mg/mL);
[49] the separation method according to any one of the preceding items [1] to [48], wherein the protein concentration of the collected or recovered fraction of the non-colored proteins is an arbitrary concentration in the range of from about 20 to about 300 mg/mL;
[50] the separation method according to any one of the preceding items [1] to [48], wherein the protein concentration of the collected or recovered fraction of the non-colored proteins is an arbitrary concentration in the range of from about 100 to about 300 mg/mL;
[51] the separation method according to any one of the preceding items [1] to [50], wherein the protein is an antibody or antibody fragment thereof;
[52] the separation method according to the preceding item [51], wherein the antibody is a bispecific antibody;
[53] the separation method according to the preceding item [51] or [52], wherein the isotype of the antibody is IgGi or IgG₄;
[54] the separation method according to the preceding item [52] or [53], wherein the bispecific antibody is a bispecific antibody capable of specifically binding to PD-1 and CD3, respectively (hereinafter, may being abbreviated as "anti-PD-1/CD3 bispecific antibody");
[55] the separation method according to the preceding item [54], wherein the anti-PD-1/CD3 bispecific antibody is the PD-1/CD3 bispecific antibody disclosed in the patent application specification identified by WO2019/156199;
[56] the separation method according to the preceding item [55], wherein the anti-PD-1/CD3 bispecific antibody comprises a heavy chain and light chain constituting an antigen-binding site specifically binding to PD-1, and a heavy chain and light chain constituting an antigen-binding site specifically binding to CD3, and wherein (a) the heavy chain of the heavy chain and light chain constituting an antigen-binding site specifically binding to PD-1 comprises the amino acid sequence set forth in any one selected from SEQ ID No. 1, SEQ ID No. 2, SEQ ID No. 3, SEQ ID No. 4 and SEQ ID No. 5, (b) the heavy chain of the heavy chain and light chain constituting an antigen-binding site specifically binding to CD3 comprises the amino acid sequence set forth in SEQ ID No. 6, and (c) both of the light chain of the heavy chain and light chain constituting an antigen-binding site specifically binding to PD-1 and the light chain of the heavy chain and light chain constituting an antigen-binding site specifically binding to CD3 comprise the amino acid sequence set forth in SEQ ID No. 7 (hereinafter, one or two or more kinds of these bispecific antibodies may be collectively abbreviated as "anti-PD-1/CD3 bispecific antibody A");
[57] the separation method according to the preceding item [56], wherein the heavy chain of the heavy chain and light chain constituting an antigen-binding site specifically binding to PD-1comprises the amino acid sequence set forth in SEQ ID No. 5;
[58] the separation method according to the preceding item [56] or [57], wherein a heavy chain constant region in each heavy chain of the heavy chain and light chain constituting an antigen-binding site specifically binding to PD-1, described in the preceding item [56] or [57], is substituted with a heavy chain constant region comprising the amino acid sequence set forth in any one selected from SEQ ID No. 8, SEQ ID No. 9, SEQ ID No. 10, SEQ ID No. 11, SEQ ID No. 12 and SEQ ID No. 13, and a heavy chain constant region in each heavy chain of the heavy chain and light chain constituting an antigen-binding site specifically binding to CD3, described in the preceding item [56] or [57], is substituted with a heavy chain constant region comprising the amino acid sequence set forth in any one selected from SEQ ID No. 14, SEQ ID No. 15, SEQ ID No. 16, SEQ ID No. 17, SEQ ID No. 18 and SEQ ID No. 19 (hereinafter, one or two of more kinds of the bispecific antibodies of which two heavy chain constant regions of the anti-PD-1/CD3 bispecific antibody A described in the preceding item [56] or [57] are substituted with the heavy chain constant regions of this item, respectively, may be collectively abbreviated as "anti-PD-1/CD3 bispecific antibody B");
[59] a method for separating non-colored proteins from a solution containing non-colored proteins and colored proteins, (1) wherein the separation method comprises
   (i) a step for loading the solution onto an anion-exchange carrier column, and using an equilibration buffer at a predetermined initial pH, making the non-colored proteins and colored proteins bind to anion-exchange carriers thereof,
   (ii) a step for flowing an elution buffer, forming a pH gradient which descends from the initial pH to a terminal pH, through the anion-exchange carrier column at a predetermined flow rate, under a condition predetermined to separate the non-colored proteins and colored proteins; and
   (iii) a step for collecting a predetermined fraction containing the non-colored proteins eluted by the preceding step (ii),
      (2) wherein the protein is a bispecific antibody specifically binding to PD-1 and CD3, respectively, comprising a heavy chain and light chain constituting an antigen-binding site specifically binding to PD-1 and a heavy chain and light chain constituting an antigen-binding site specifically binding to CD3, and wherein (a) the heavy chain of the heavy chain and light chain constituting an antigen-binding site specifically binding to PD-1 comprises the amino acid sequence set forth in any one selected from SEQ ID No. 1, SEQ ID No. 2, SEQ ID No. 3, SEQ ID No. 4 and SEQ ID No. 5, (b) the heavy chain of the heavy chain and light chain constituting an antigen-binding site specifically binding to CD3 comprises the amino acid sequence set forth in SEQ ID No. 6, and (c) both of the light chain of the heavy chain and light chain constituting an antigen-binding site specifically binding to PD-1 and the light chain of the heavy chain and light chain constituting an antigen-binding site specifically binding to CD3 comprise the amino acid sequence set forth in SEQ ID No. 7,
      (3) wherein the anion-exchange carrier is Toyopearl (registered trademark) NH2-750F,
      (4) wherein the initial pH of the equilibration buffer and elution buffer is an arbitrary pH in the range of from about 10.6 to about 8.4, and the terminal pH of the elution buffer is an arbitrary pH in the range of from about 7.0 to about 4.0 (provided that the initial pH is at least 2.0 higher than the terminal pH),
      (5) wherein the pH gradient is performed (a) by increasing the percentage (%) of the buffer B in the elution buffer step-by-step by from about 1 to about 2 %, at an interval corresponding to an arbitrary column volume in the range of from about 4 to about 6 CV, over at least the range of from about 50 to 100%, or (b) by decreasing the pH of the elution buffer step-by-step by an arbitrary pH in the range of from about 0.6 to about 1.0, within a range corresponding to the column volume of from about 30 to about 40 CV,
      (6) wherein the linear flow rate of the elution buffer is an arbitrary linear flow rate from about 100 to about 800 cm/h, and
      (7) wherein the color concentration of the collected solution containing the non-colored proteins is (i) substantially the same as or lighter than that of BY5 in the visual comparison test with the reference standard solution BY5, or (ii) the Em/UV*100 value of the collected solution containing the non-colored proteins is about 6.3 or less;
[60] a method for separating non-colored proteins from a solution containing non-colored proteins and colored proteins,
   (1) wherein the separation method comprises
      (i) a step for loading the solution onto an anion-exchange carrier column,
      (ii) a step for flowing an equilibration buffer prepared so that the colored proteins in the solution bind to anion-exchange carriers thereof and the non-colored proteins do not, through the anion-exchange carrier column at a predetermined flow rate, and
      (iii) a step for collecting the fraction containing the non-colored proteins flowed through in the preceding step (ii) as it is,
   (2) wherein the protein is a bispecific antibody specifically binding to PD-1 and CD3, respectively, comprising a heavy chain and light chain constituting an antigen-binding site specifically binding to PD-1 and a heavy chain and light chain constituting an antigen-binding site specifically binding to CD3, and wherein (a) the heavy chain of the heavy chain and light chain constituting an antigen-binding site specifically binding to PD-1 comprises the amino acid sequence set forth in any one selected from SEQ ID No. 1, SEQ ID No. 2, SEQ ID No. 3, SEQ ID No. 4 and SEQ ID No. 5, (b) the heavy chain of the heavy chain and light chain constituting an antigen-binding site specifically binding to CD3 comprises the amino acid sequence set forth in SEQ ID No. 6, and (c) both of the light chain of the heavy chain and light chain constituting an antigen-binding site specifically binding to PD-1 and the light chain of the heavy chain and light chain constituting an antigen-binding site specifically binding to CD3 comprise the amino acid sequence set forth in SEQ ID No. 7,
   (3) wherein the anion-exchange carrier is Toyopearl (registered trademark) NH2-750F,
   (4) wherein the pH of the equilibration buffer is an arbitrary pH in the range of from about 8.2 to about 7.8, and the electrical conductivity is an arbitrary electrical conductivity from about 5 to about 7 mS/cm,
   (5) wherein the linear flow rate of the equilibration buffer is an arbitrary linear flow rate in the range of from about 100 to about 400 cm/h, and
   (6) wherein the color concentration of the collected solution containing the non-colored proteins is (i) substantially the same as or lighter than that of BY5 in the visual comparison test with the reference standard solution BY5, or (ii) the Em/UV*100 value of the collected solution containing the non-colored protein is about 6.3 or less;

[1-1] a method for preparing a solution containing a non-colored protein (preferably, a noncolorant of the antibody, bispecific antibody or anti-PD-1/CD3 bispecific antibody described in any one of the preceding items [51] to [58]), which comprises the separation method (separation process) described in any one of the preceding items [1] to [60];
[2-1] a colorless or slightly colored solution containing the anti-PD-1/CD3 bispecific antibody described in any one of the preceding items [55] to [58], at a concentration of from about 100 to about 300 mg/mL;
[2-2] the solution according to the preceding item [2-1], wherein the concentration of the anti-PD-1/CD3 bispecific antibody is within the range of from about 100 to about 200 mg/mL;
[2-3] the solution according to the preceding item [2-1], wherein the concentration of the anti-PD-1/CD3 bispecific antibody is within the range of from about 100 to about 150 mg/mL;
[2-4] the solution according to the preceding item [2-1], wherein the concentration of the anti-PD-1/CD3 bispecific antibody is within the range of from about 100 to about 120 mg/mL;
[2-5] the solution according to any one of the preceding items [2-1] to [2-4], except for solutions which are colorless or slightly colored without any process to reduce or remove the coloration;
[2-6] a colorless or slightly colored solution containing the anti-PD-1/CD3 bispecific antibody described in any one of the preceding items [55] to [58] and prepared by processes including the separation method described in any one of the preceding items [1] to [60];
[2-7] the solution according to any one of the preceding items [2-1] to [2-5], prepared by processes including the separation method described in any one of the preceding items [1] to [60];
[2-8] the solution according to any one of the preceding items [2-1] to [2-7], prepared by being further concentrated as necessary;
[2-9] the solution according to any one of the preceding items [2-1] to [2-8], which is substantially the same as or lighter than BY5 in the visual comparison test with the reference standard solution BY5;
[2-10] the solution according to any one of the preceding items [2-1] to [2-8], which is substantially the same as or lighter than BY6 in the visual comparison test with the reference standard solution BY6;
[2-11] the solution according to any one of the preceding items [2-1] to [2-8], which is substantially the same as BY7 in the visual comparison test with the reference standard solution BY7;
[2-12] the solution according to any one of the preceding items [2-1] to [2-8], of which the Em/UV*100 value is about 6.3 or less;
[2-13] the solution according to any one of the preceding items [2-1] to [2-8] and [2-10], of which the Em/UV*100 value is about 4.1 or less;
[3-1] a pharmaceutical composition containing the solution described in any one of the preceding items [2-1] to [2-13]; and
[4-1] use of the pharmaceutical composition described in the preceding item [3-1], in preventing, suppressing the symptom progression of, suppressing the recurrence of, and/or treating autoimmune disease, graft-versus-host disease (GVHD) or hematological cancer.

### [Advantage Effects of Invention]

The method of the present invention for removing the coloration of drug substance of protein preparation makes it possible to remove coloration of protein solution caused by advanced glycation end products.

### [Brief Description of the Drawings]

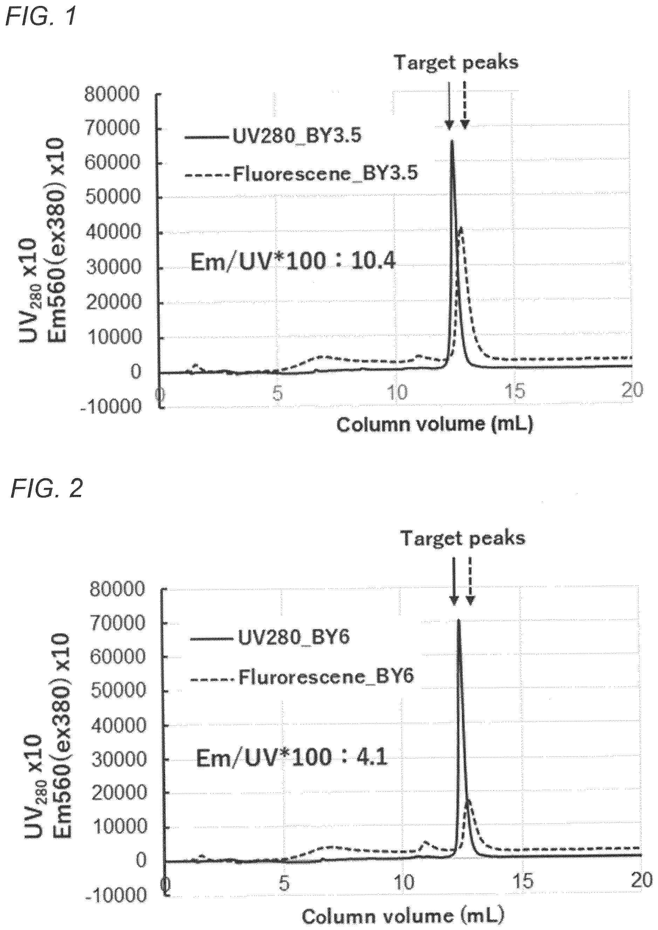

[Figure 1] It shows a reversed-phase HPLC analysis chromatogram of a sample prepared by fractionating anti-PD-1/CD3 bispecific antibody by strong cation-exchange chromatography, concentrating the fractions to 100 mg/mL, and then mixing the high and low colored fractions thereof so as to correspond to the reference standard solution BY3.5. The X-axis represents the column volume (mL), and the Y-axis represents the value calculated by multiplying the peak area of UV absorbance (UV₂₈₀) and the peak area of fluorescence intensity (Em560(ex380)) by 10 (UV₂₈₀×10, Em560(ex380)×10), respectively. In the figure, the solid line represents the UV chromatogram (UV280_BY3.5) and the dashed line represents the fluorescence chromatogram (Fluorescene_BY3.5). The arrows indicate the peaks of targets.
[Figure 2] It shows a reversed-phase HPLC analysis chromatogram of a sample prepared by fractionating anti-PD-1/CD3 bispecific antibody by strong cation-exchange chromatography, concentrating the fractions to 100 mg/mL, and then mixing the high and low colored fractions thereof so as to correspond to the reference standard solution BY6.0. The X-axis represents the column volume (mL), and the Y-axis represents the value calculated by multiplying the peak area of UV absorbance (UV₂₈₀) and the peak area of fluorescence intensity (Em560(ex380)) by 10 (UV₂₈₀×10, Em560(ex380)×10), respectively. In the figure, the solid line represents the UV chromatogram (UV280_BY6.0) and the dashed line represents the fluorescence chromatogram (Fluorescene_BY6.0). The arrows indicate the peaks of targets.

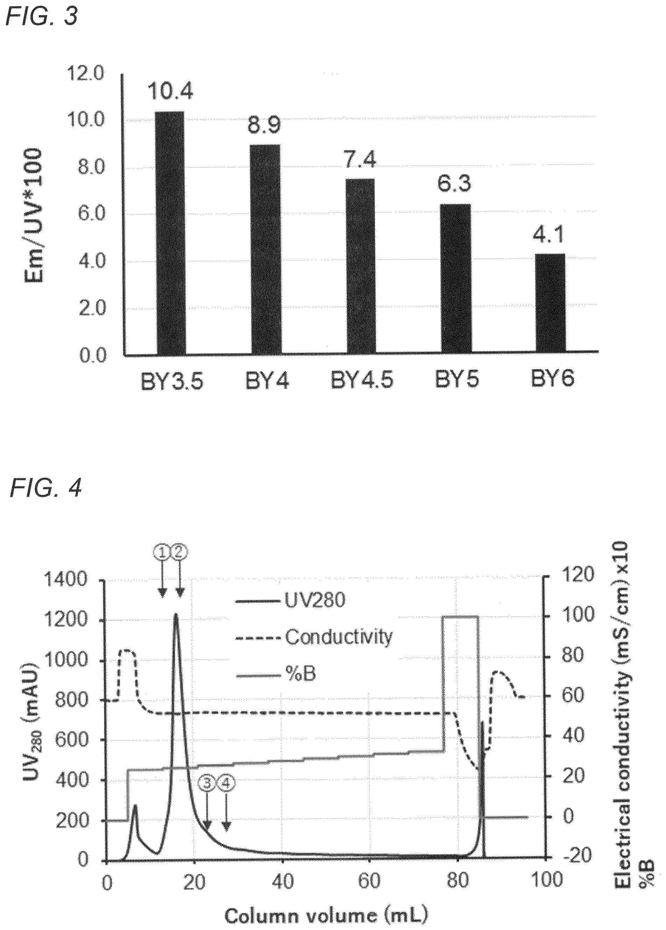

[Figure 3] It shows the results of HPLC analysis of samples, prepared by mixing the high and low colored fractions of anti-PD-1/CD3 dual specificity antibody concentrated to 100 mg/mL, and compared to the reference standard solutions BY3.5, BY4, BY4.5, BY5 and BY6, respectively. The horizontal axis represents the number of the reference standard solution, the vertical axis represents the Em/UV*100 value, and the numerical value at the top of each vertical bar graph represents the Em/UV* 100 value of each reference standard solution.
[Figure 4] It shows a pH step-wise gradient elution chromatogram in the Bind-elute mode of anion-exchange using a column packed with Toyopearl (registered trademark) NH2-750F. The sample used for the separation operation is an antibody. The X-axis represents the column volume (mL), the left Y-axis represents the peak area of UV absorption (UV₂₈₀), and the right Y-axis represents the value calculated by multiplying the electrical conductivity (mS/cm) by 10. The right Y-axis also indicates the percentage of liquid value (%B) pumped by B pump among the two mobile phase pumps A and B. In the figure, the solid black line represents the UV chromatogram (UV280), the dashed line represents the electrical conductivity (Conductivity), and the solid gray line represents the percentage of liquid (%B) pumped by B pump. The arrows with the numbers 1 to 4 indicate the positions of the purified fractions used for colorimetric analysis.

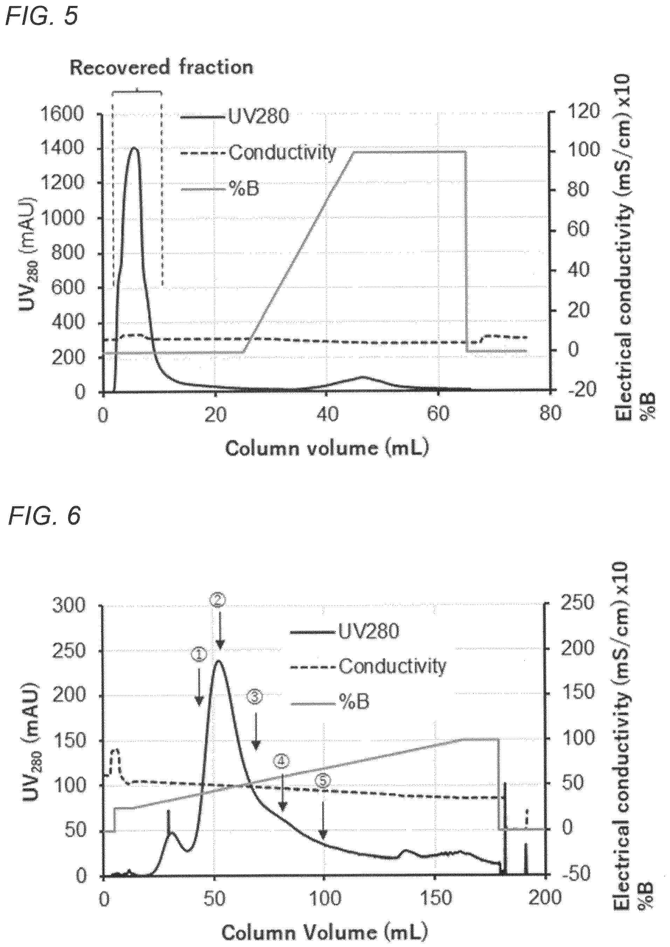

[Figure 5] It shows a chromatogram in the flow-through mode of anion-exchange using a column packed with Toyopearl (registered trademark) NH2-750F. The sample used for the separation operation is an antibody. The x- and y-axes, as well as the solid black, dashed and solid gray lines in the figure have the same meaning as those in Figure 4, respectively. The inverted U represents the range of recovered fractions analyzed after purification.
[Figure 6] It shows a pH linear gradient elution chromatogram in the Bind-elute mode of anion-exchange using a column packed with Capto (registered trademark) adhere. The sample used for the separation operation is an antibody. The x- and y-axes, as well as the solid black, dashed and solid gray lines in the figure have the same meaning as those in Figure 4, respectively. The arrows with the numbers 1 to 5 indicate the positions of purified fractions for colorimetric analysis.

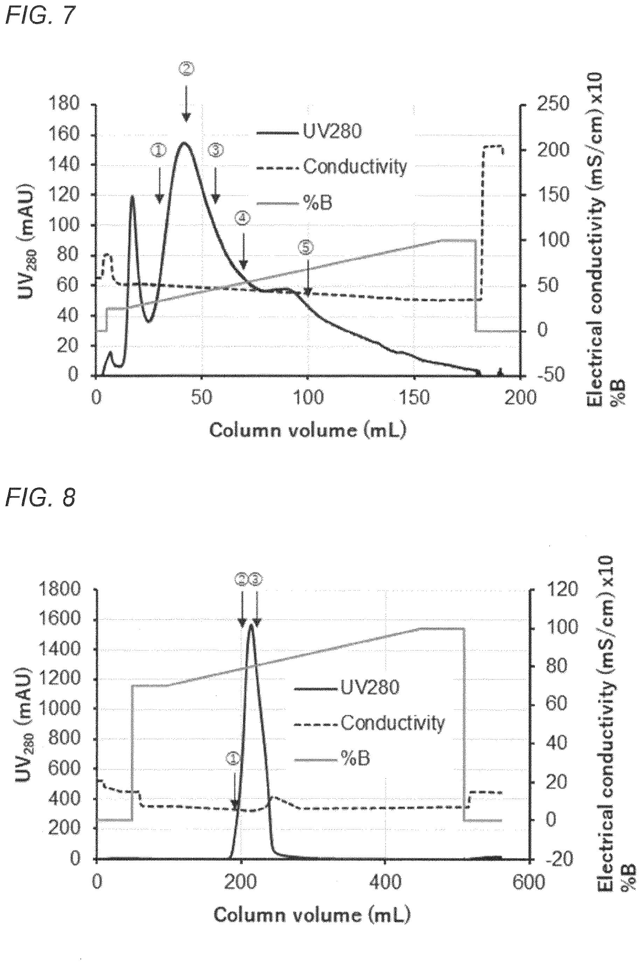

[Figure 7] It shows a pH linear gradient elution chromatogram in the Bind-elute mode of anion-exchange using a column packed with Cellufine MAX IB. The sample used for the separation operation is an antibody. The x- and y-axes, as well as the solid black, dashed and solid gray lines in the figure have the same meaning as those in Figure 4, respectively. The arrows with the numbers 1 to 5 indicate the positions of purified fractions for colorimetric analysis.
[Figure 8] It shows a pH linear gradient elution chromatogram in the Bind-elute mode of anion-exchange using a column packed with Capto (registered trademark) Q. The sample used for the separation operation is bovine serum-derived albumin (BSA). The x- and y-axes, as well as the solid black, dashed and solid gray lines in the figure have the same meaning as those in Figure 4, respectively. The arrows with the numbers 1 to 3 indicate the positions of purified fractions for colorimetric analysis.

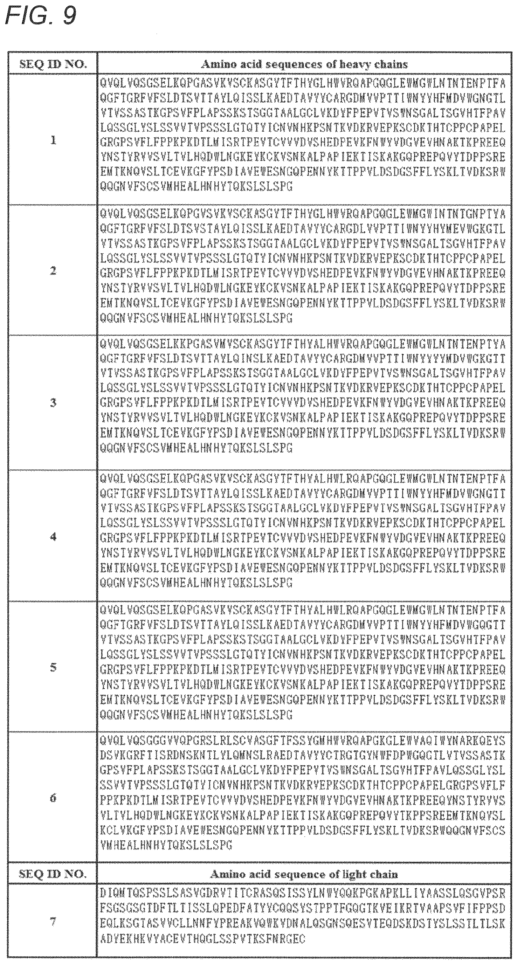

[Figure 9] It shows the respective amino acid sequences of the heavy chain and light chain constituting the antigen-binding site specifically binding to PD-1 and the heavy chain and light chain constituting the antigen-binding site specifically binding to CD3, constituting the anti-PD-1/CD3 bispecific antibody A.

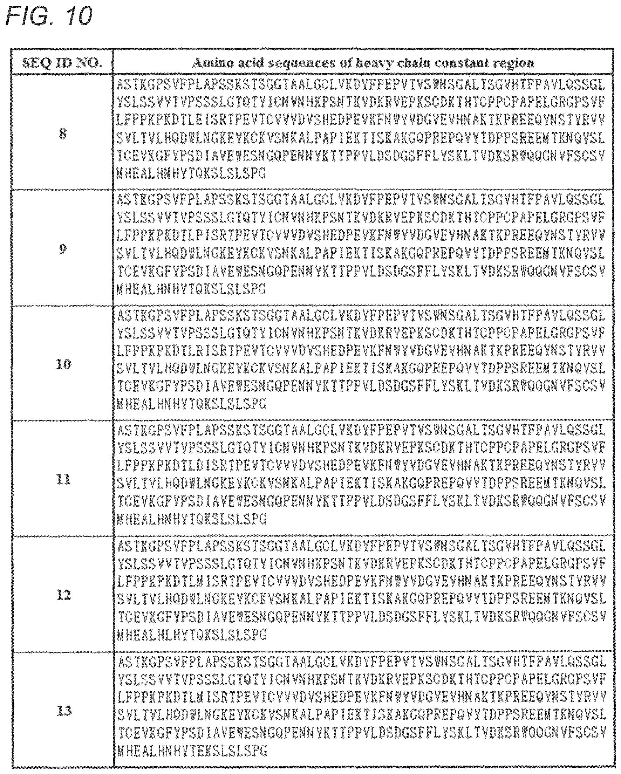

[Figure 10] It shows the respective amino acid sequences of the heavy chain constant regions (the heavy chain constant regions in the heavy chain and light chain constituting the antigen-binding sites specifically binding to PD-1), constituting the anti-PD-1/CD3 bispecific antibody B.

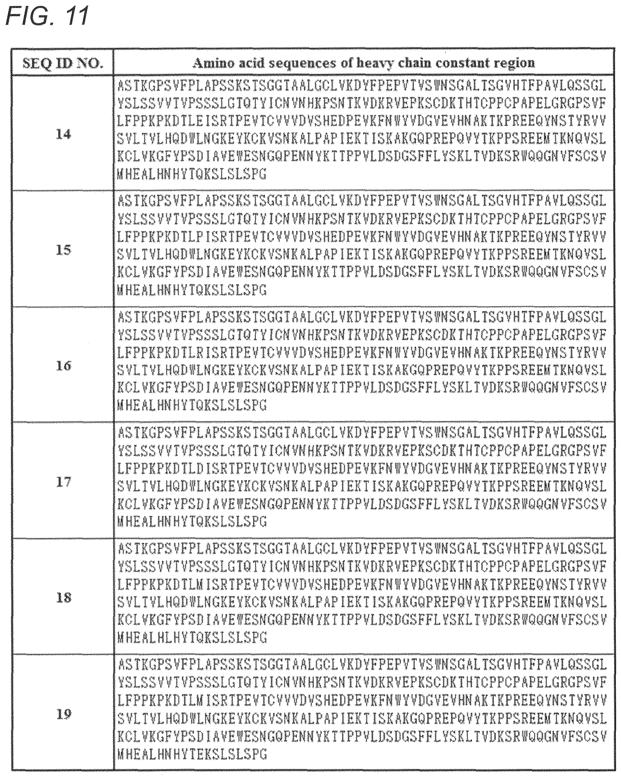

[Figure 11] It shows the respective amino acid sequences of the heavy chain constant regions (the heavy chain constant regions in the heavy chain and light chain constituting the antigen-binding sites specifically binding to CD3), constituting the anti-PD-1/CD3 bispecific antibody B.

### [Description of Embodiments]

The present inventions relate to the method for separating non-colored proteins from a solution containing non-colored proteins and colored proteins, wherein the separation method (hereinafter, "Bind-Elute method") comprises
(1) the step for loading the solution onto an ion-exchange carrier column, and using an equilibration buffer at a predetermined initial pH, making the non-colored proteins and colored proteins bind to the ion-exchange carrier,
(2) the step for flowing an elution buffer, forming the pH gradient varying from the initial pH to the terminal pH, through the ion-exchange carrier column at the predetermined flow rate, under conditions predetermined to separate the non-colored proteins and colored proteins, and
(3) the step for collecting the predetermined fraction containing the non-colored proteins eluted by the preceding step (2).

Furthermore, the present inventions include the method for separating non-colored proteins from the solution containing non-colored proteins and colored proteins, wherein the separation method (hereinafter, "Flow-Through method") comprises
(1a) the step for loading the solution onto the ion-exchange carrier column,
(2a) the step for flowing an equilibration buffer prepared so that the colored proteins in the solution bind to the ion-exchange carrier and the non-colored proteins do not, through the ion-exchange carrier column at the predetermined flow rate, and
(3a) the step for collecting the fraction containing the non-colored proteins flowed through in the preceding step (2a) as it is.

The "colored protein" in the present invention is a modified protein causing the coloration which is the objection of the present invention, and the modified protein causing the coloration include several types of Advanced Glycation End products (hereinafter, may be abbreviated as "AGEs"), and the solutions containing these AGEs have light yellow or yellow color, depending on the amount of AGEs produced. Herein, the term "Advanced Glycation End products" is a generic term for several types of modified proteins which are produced repeatedly by non-enzymatic reaction of free amino groups on lysine residues or the like of proteins with reducing sugars such as glucose and fructose, followed by reactions such as irreversible dehydration, condensation, oxidation and/or reduction, and the formations of various AGEs such as glucose derived AGEs, glyceraldehyde derived AGEs, glycolaldehyde derived AGEs, methylglyoxal derived AGEs, glyoxal derived AGEs and 3-deoxyglucosone derived AGEs have been confirmed, and it has been reported that some of these AGEs contain a yellowish-brown fluorescent modification. The isoelectric point of the colored protein is within the range of from about 5 to about 9.1, and generally within the range of from about 6 to about 7.

On the other hand, the "non-colored protein" is a purposed protein in which the protein modification has not substantially occurred or is so light that its solution is recognized as not visually colored. The isoelectric point of the non-colored protein falls within the range of from about 7 to about 9.5, specifically, within any range selected from the group consisting of about 7.0 or more but less than about 7.5, about 7.5 or more but less than about 8.0, about 8.0 or more but less than about 8.5, about 8.5 or more but less than about 9.0, and about 9.0 or more but than about 9.5.

Examples of the protein solutions to which the separation method of the present invention is required to be applied include those of which the color concentration of itself or concentrated solution thereof is more than that of BY5 in the visual comparison test with the reference standard solution BY5 in item 2.65 "color comparison test" in the general test of the Japanese Pharmacopoeia, 17th revision, or the Em/UV^{∗}100 value is more than about 6.3.

The "equilibration buffer at a predetermined initial pH" in the present invention is a buffer with a pH value which is substantially the same as a pH value predetermined as the upper limit of the pH gradient formed by the elution buffer used in the present invention, and can be prepared in advance to achieve a predetermined electrical conductivity and pH value at which both of the non-colored proteins and colored proteins in solution loaded into an ion-exchange carrier column can bind to an ion-exchange carrier thereof.

The electrical conductivity of the equilibration buffer and/or elution buffer used in the separation method of the present invention (Bind-Elute method) can be predetermined to be an optimum electrical conductivity, depending on the nature of the protein applied and the type of the anion-exchange carrier concerned. For example, an arbitrary electrical conductivity in the range of from about 1 to about 20 mS/cm can be selected, preferably an arbitrary electrical conductivity in the range of from about 2 to about 15 mS/cm, and specifically, examples thereof include about 2 mS/cm, about 3 mS/cm, about 4 mS/cm, about 5 mS/cm, about 6 mS/cm, about 7 mS/cm, about 8 mS/cm, about 9 mS/cm, about 10 mS/cm, about 11 mS/cm, about 12 mS/cm, about 13 mS/cm, about 14 mS/cm and about 15 mS/cm. In the case of the Bind-Elute method, generally, the electrical conductivities of the equilibration buffer and the elution buffer are substantially the same.

The pH and electrical conductivity of the equilibration buffer used in the separation method of the present invention (Flow-Through method) can be prepared in advance so that the colored proteins in solution bind to the ion-exchange carrier and the non-colored proteins in solution do not bind to the carrier, and the pH can be an arbitrary pH value from about 9.2 to about 7.4, specifically, examples thereof include about 9.2, about 9.0, about 8.8, about 8.6, about 8.4, about 8.2, about 8.0, about 7.8, about 7.6 and about 7.4, preferably an arbitrary pH value in the range of from about 8.6 to about 7.4, and more preferably an arbitrary pH value in the range of from about 8.2 to about 7.4, and even more preferably an arbitrary pH value in the range of from about 8.2 to about 7.8. On the other hand, an arbitrary electrical conductivity in the range of from about 1 to about 20 mS/cm can be selected, and preferably an arbitrary electrical conductivity in the range of from about 2 to 15 mS/cm, specifically, examples thereof include 2 mS/cm, about 3 mS/cm, about 4 mS/cm, about 5 mS /cm, about 6 mS/cm, about 7 mS/cm, about 8 mS/cm, about 9 mS/cm, about 10 mS/cm, about 11 mS/cm, about 12 mS/cm, about 13 mS/cm, about 14 mS/cm, and about 15 mS/cm, and preferably an arbitrary electrical conductivity in the range of from about 5 to 7 mS/cm. For example, as the equilibration buffer used in the Flow-Through method, HEPES buffer (pH 8.0) is preferred.

The washing buffer used in the separation method of the present invention is mainly used to wash the anion-exchange carrier prior to elution of the non-colored proteins. Examples of the washing buffers used include one having a composition which can remove impurities other than the target non-colored proteins and colored proteins from the ion-exchange carrier, or one having a high pH or low electrical conductivity well outside the elution range of the target non-colored proteins.

The ion-exchange carrier in the present invention is preferably an anion-exchange carrier. Examples of forms thereof include that of small particle, membrane or monolith, but preferably that of small particle. The ion-exchange carrier column in the present invention refers to a column packed with an ion-exchange carrier.

Herein, the anion-exchange carrier is not limited, as long as exhibiting anion-exchange effects, and examples thereof include a strong anion-exchange carrier, weak anion-exchange carrier and multi-mode anion-exchange carrier.

Examples of the strong anion-exchange carriers include Fractogel (registered trademark) EMD TMAE (M), Fractogel (registered trademark) EMD TMAE Medcap (M), Fractogel (registered trademark) EMD TMAE Hicap (M), Eshmuno (registered trademark) Q, Eshmuno (registered trademark) QPX, Eshmuno (registered trademark) QPX Hicap, Capto (registered trademark) Q, Capto (registered trademark) Q ImpRes, Q Sepharose (registered Trademark) FF, Q Sepharose (registered trademark) HP, Q Sepharose (registered trademark) XL, Source (Registered (registered trademark) 30Q, POROS (registered trademark) 50 HQ, POROS (registered trademark) 50 XQ, POROS (registered trademark) 50 PI, Q HyperCel (registered trademark), Toyopearl (registered trademark) GigaCap Q 650M, Toyopearl (registered trademark) GigaCap Q 650S, Toyopearl (registered trademark) Super Q, YMC (registered trademark) BioPro Q, Macro-Prep (registered trademark) High Q, Nuvia (registered trademark) Q, UNOsphere (registered trademark) Q, Cellufine (registered trademark) Q-500, Cellufine (registered trademark) MAX Q-r and Cellufine (registered trademark) MAX Q-h.

Examples of the weak anion-exchange carriers include Fractogel (registered trademark) EMD DEAE, Fractogel (registered trademark) EMD DMAE, Capto (registered trademark) DEAE, DEAE Ceramic HyperD (registered trademark) F, Toyopearl (registered trademark) NH2-750 F, TOYOPEARL (registered trademark) DEAE-650C, TOYOPEARL (registered trademark) DEAE-650M, TOYOPEARL (registered trademark) DEAE-650S, Cellufine (registered trademark) A-200, Cellufine (registered trademark) A-500, Cellufine (registered trademark) A-800, and Cellufine (registered trademark) MAX DEAE.

Examples of the multi-mode anion-exchange carriers include Toyopearl (registered trademark) NH2-750F, Capto (registered trademark) Adhere, Capto (registered trademark) Adhere ImpRes, Capto (registered trademark) MMC, Capto (registered trademark) core 700 and Cellufine (registered trademark) IB.

Herein, the anion-exchange carrier that can be used in the separation method of the present invention is preferably a multi-mode anion-exchange carrier, and more preferably Toyopearl (registered trademark) NH2-750F.

The "elution buffer forming a pH gradient varying from the initial pH to the terminal pH" in the present invention is an eluent consisting of a combination of two or more buffers prepared to form a pH gradient varying in a linear gradient or step-wise gradient over a pH range interposing at least the isoelectric point of the target protein to be eluted. The elution buffer can be prepared, for example, by predetermining the pH range according to the isoelectric point of the target protein and distribution thereof, and combining the buffer at the initial pH, which is the upper limit of the pH range (hereinafter, may be abbreviated as "buffer A"), and the buffer at the terminal pH, which is the lower limit of the pH range (hereinafter, may be abbreviated as "buffer B"), at an arbitrary ratio so as to achieve a predetermined pH, and the pH gradient in the process can be formed by varying the combination ratio of the two buffers continuously or step-wise.

The pH gradient applied in the separation method of the present invention is a pH gradient which descends from the initial pH to the terminal pH. Herein, the initial pH is, for example, an arbitrary pH value in the range of from about 11.0 to about 7.0, specifically, any pH value selected from the group consisting of about 11.0, about 10.8, about 10.6, about 10.4, about 10.2, about 10.0, about 9.8, about 9.6, about 9.4, about 9.2, about 9.0, about 8.8, about 8.6, about 8.4, about 8.2, about 8.0, about 7.8, about 7.6, about 7.4, about 7.2 and about 7.0, preferably an arbitrary pH value in the respective ranges of from about 10.8 to about 7.2, from about 10.8 to about 7.4, from about 10.8 to about 7.6, from about 10.8 to about 7.8, from about 10.8 to about 8.0, from about 10.8 to about 8.2, from about 10. 8 to about 8.4, from about 10.6 to about 7.2, from about 10.6 to about 7.4, from about 10.6 to about 7.6, from about 10.6 to about 7.8, from about 10.6 to about 8.0, from about 10.6 to about 8.2, or from about 10.6 to about 8.4, and more preferably an arbitrary pH value in the range of from about 10.6 to about 8.4. On the other hand, the terminal pH is, for example, an arbitrary pH value in the range of from about 4.0 to about 8.2, specifically, any pH value selected from the group consisting of about 4.0, about 4.2, about 4.4, about 4.6, about 4.8, about 5.0, about 5.2, about 5.4, about 5.6, about 5.8, about 6.0, about 6.2, about 6.4, about 6.6, about 6.8, about 7.0, about 7.2, about 7.4, about 7.6, about 7.8, about 8.0 and about 8.2, and preferably, an arbitrary pH value in the respective ranges of from about 4.0 to about 8.0, from about 4.0 to about 7.8, from about 4.0 to about 7.6, from about 4.0 to about 7.4, from about 4.0 to about 7.2, or from about 4.0 to about 7.0, more preferably, an arbitrary pH value in the range of from about 4.0 to about 7.0. However, the initial pH is at least about 1.0 higher than the terminal pH.

Furthermore, examples of the pH gradient ranges include the range between any one of the initial pH selected from the group consisting of about 11.0, about 10.8, about 10.6, about 10.4, about 10.2, about 10.0, about 9.8, about 9.6, about 9.4, about 9.2, about 9.0, about 8.8, about 8.6, about 8.4, about 8.2, about 8.0, about 7.8, about 7.6, about 7.4, about 7.2 and about 7.0 and any one of the terminal pH selected from the group consisting of about 4.0, about 4.2, about 4.4, about 4.6, about 4.8, about 5.0, about 5.2, about 5.4, about 5.6, about 5.8, about 6.0, about 6.2, about 6.4, about 6.6, about 6.8, about 7.0, about 7.2, about 7.4, about 7.6, about 7.8, about 8.0 and about 8.2, preferably, the initial pH is at least 2.0 higher than the terminal pH. Specific examples of the pH gradient ranges include from about 11.0 to about 4.0, from about 11.0 to about 4.2, from about 11.0 to about 4.4, from about 11.0 to about 4.6, from about 11.0 to about 4.8, from about 11.0 to about 5.0, from about 11.0 to about 5.2, from about 11.0 to about 5.4, from about 11.0 to about 5.6, from about 11.0 to about 5.8, from about 11.0 to about 6.0, from about 11.0 to about 6.2, from about 11.0 to about 6.4, from about 11.0 to about 6.6, from about 11.0 to about 6.8, from about 11.0 to about 7.0, from about 11.0 to about 7.2, from about 11.0 to about 7.4, from about 11.0 to about 7.6, from about 11.0 to about 7.8, from about 11.0 to about 8.0, from about 11.0 to about 8.2, from about 10.8 to about 4.0, from about 10.8 to about 4.2, from about 10.8 to about 4.4, from about 10.8 to about 4.6, from about 10.8 to about 4.8, from about 10.8 to about 5.0, from about 10.8 to about 5.2, from about 10.8 to about 5.4, from about 10.8 to about 5.6, from about 10.8 to about 5.8, from about 10.8 to about 6.0, from about 10.8 to about 6.2, from about 10.8 to about 6.4, from about 10.8 to about 6.6, from about 10.8 to about 6.8, from about 10.8 to about 7.0, from about 10.8 to about 7.2, from about 10.8 to about 7.4, from about 10.8 to about 7.6, from about 10.8 to about 7.8, from about 10.8 to about 8.0, from about 10.8 to about 8.2, from about 10.6 to about 4.0, from about 10.6 to about 4.2, from about 10.6 to about 4.4, from about 10.6 to about 4.6, from about 10.6 to about 4.8, from about 10.6 to about 5.0, from about 10.6 to about 5.2, from about 10.6 to about 5.4, from about 10.6 to about 5.6, from about 10.6 to about 5.8, from about 10.6 to about 6.0, from about 10.6 to about 6.2, from about 10.6 to about 6.4, from about 10.6 to about 6.6, from about 10.6 to about 6.8, from about 10.6 to about 7.0, from about 10.6 to about 7. 2, from about 10.6 to about 7.4, from about 10.6 to about 7.6, from about 10.6 to about 7.8, from about 10.6 to about 8.0, from about 10.6 to about 8.2, from about 10.4 to about 4.0, from about 10.4 to about 4.2, from about 10.4 to about 4.4, from about 10.4 to about 4.6, from about 10.4 to about 4.8, from about 10.4 to about 5.0, from about 10.4 to about 5.2, from about 10.4 to about 5.4, from about 10.4 to about 5.6, from about 10.4 to about 5.8, from about 10.4 to about 6.0, from about 10.4 to about 6.2, from about 10.4 to about 6.4, from about 10.4 to about 6. 6, from about 10.4 to about 6.8, from about 10.4 to about 7.0, from about 10.4 to about 7.2, from about 10.4 to about 7.4, from about 10.4 to about 7.6, from about 10.4 to about 7.8, from about 10.4 to about 8.0, from about 10.4 to about 8.2, from about 10.2 to about 4.0, from about 10.2 to about 4.2, from about 10.2 to about 4.4, from about 10.2 to about 4.6, from about 10.2 to about 4.8, from about 10.2 to about 5.0, from about 10.2 to about 5.2, from about 10.2 to about 5.4, from about 10.2 to about 5.6, from about 10.2 to about 5.8, from about 10.2 to about 6.0, from about 10.2 to about 6.2, from about 10.2 to about 6.4, from about 10.2 to about 6.6, from about 10.2 to about 6.8, from about 10.2 to about 7.0, from about 10.2 to about 7.2, from about 10.2 to about 7.4, from about 10.2 to about 7.6, from about 10.2 to about 7.8, from about 10.2 to about 8.0, from about 10.2 to about 8.2, from about 10.0 to about 4.0, from about 10.0 to about 4.2, from about 10.0 to about 4.4, from about 10.0 to about 4.6, from about 10.0 to about 4.8, from about 10.0 to about 5.0, from about 10.0 to about 5.2, from about 10.0 to about 5.4, from about 10.0 to about 5.6, from about 10.0 to about 5.8, from 10.0 to about 6.0, from about 10.0 to about 6.2, from about 10.0 to about 6.4, from about 10.0 to about 6.6, from about 10.0 to about 6.8, from about 10.0 to about 7.0, from about 10.0 to about 7.2, from about 10.0 to about 7.4, from about 10.0 to about 7.6, from about 10.0 to about 7.8, from about 10.0 to about 8.0, from about 9.8 to about 4.0, from about 9.8 to about 4.2, from about 9.8 to about 4.4, from about 9.8 to about 4.6, from about 9.8 to about 4.8, from about 9.8 to about 5.0, from about 9.8 to about 5.2, from about 9.8 to about 5.4, from about 9.8 to about 5.6, from about 9.8 to about 5.8, from about 9.8 to about 6.0, from about 9.8 to about 6.2, from about 9.8 to about 6.4, from about 9.8 to about 6.6, from about 9.8 to about 6.8, from about 9.8 to about 7.0, from about 9.8 to about 7.2, from about 9.8 to about 7.4, from about 9.8 to about 7.6, from about 9.8 to about 7.8, from about 9.6 to about 4.0, from about 9.6 to about 4.2, from about 9.6 to about 4.4, from about 9.6 to about 4.6, from about 9.6 to about 4.8, from about 9.6 to about 5.0, from about 9.6 to about 5.2, from about 9.6 to about 5.4, from about 9.6 to about 5.6, from about 9.6 to about 5.8, from about 9.6 to about 6.0, from about 9.6 to about 6.2, from about 9.6 to about 6.4, from about 9.6 to about 6.6, from about 9.6 to about 6.8, from about 9.6 to about 7.0, from about 9.6 to about 7.2, from about 9.6 to about 7.4, from about 9.6 to about 7.6, from about 9.4 to about 4.0, from about 9.4 to about 4.2, from about 9.4 to about 4.4, from about 9.4 to about 4.6, from about 9.4 to about 4.8, from about 9.4 to about 5.0, from about 9.4 to about 5.2, from about 9.4 to about 5.4, from about 9.4 to about 5.6, from about 9.4 to about 5.8, from about 9.4 to about 6.0, from about 9.4 to about 6.2, from about 9.4 to about 6.4, from about 9.4 to about 6.6, from about 9.4 to about 6.8, from about 9.4 to about 7.0, from about 9.4 to about 7.2, from about 9.4 to about 7.4, from about 9.2 to about 4.0, from about 9.2 to about 4.2, from about 9.2 to about 4.4, from about 9.2 to about 4.6, from about 9.2 to about 4.8, from about 9.2 to about 5.0, from about 9.2 to about 5.2, from about 9.2 to about 5.4, from about 9.2 to about 5.6, from about 9.2 to about 5.8, from about 9.2 to about 6.0, from about 9.2 to about 6.2, from about 9.2 to about 6.4, from about 9.2 to about 6.6, from about 9.2 to about 6.8, from about 9.2 to about 7.0, from about 9.2 to about 7.2, from about 9.0 to about 4.0, from about 9.0 to about 4.2, from about 9.0 to about 4.4, from about 9.0 to about 4.6, from about 9.0 to about 4.8, from about 9.0 to about 5.0, from about 9.0 to about 5.2, from about 9.0 to about 5.4, from about 9.0 to about 5.6, from about 9.0 to about 5.8, from about 9.0 to about 6.0, from about 9.0 to about 6.2, from about 9.0 to about 6.4, from about 9.0 to about 6.6, from about 9.0 to about 6.8, from about 9.0 to about 7.0, from about 8.8 to about 4.0, from about 8.8 to about 4.2, from about 8.8 to about 4.4, from about 8.8 to about 4.6, from about 8.8 to about 4.8, from about 8.8 to about 5.0, from about 8.8 to about 5.2, from about 8.8 to about 5.4, from about 8.8 to about 5.6, from about 8.8 to about 5.8, from about 8.8 to about 6.0, from about 8.8 to about 6.2, from about 8.8 to about 6.4, from about 8.8 to about 6.6, from about 8.8 to about 6.8, from about 8.6 to about 4.0, from about 8.6 to about 4.2, from about 8.6 to 4.4, from about 8.6 to about 4.6, from about 8.6 to about 4.8, from about 8.6 to about 5.0, from about 8.6 to about 5.2, from about 8.6 to about 5.4, from about 8.6 to about 5.6, from about 8.6 to about 5.8, from about 8.6 to about 6.0, from about 8.6 to about 6.2, from about 8.6 to about 6.4, from about 8.6 to about 6.6, from about 8.4 to about 4.0, from about 8.4 to about 4.2, from about 8.4 to about 4.4, from about 8.4 to about 4.6, from about 8.4 to about 4.8, from about 8.4 to about 5.0, from about 8.4 to about 5.2, from about 8.4 to about 5.4, from about 8.4 to about 5.6, from about 8.4 to about 5.8, from about 8.4 to about 6.0, from about 8.4 to about 6.2, from about 8.4 to about 6.4, from about 8.2 to about 4.0, from about 8.2 to about 4.2, from about 8.2 to about 4.4, from about 8.2 to about 4.6, from about 8.2 to about 4.8, from about 8.2 to about 5.0, from about 8.2 to about 5.2, from about 8.2 to about 5.4, from about 8.2 to about 5.6, from about 8.2 to about 5.8, from about 8.2 to about 6.0, from about 8.2 to about 6.2, from about 8.0 to about 4.0, from about 8.0 to about 4.2, from about 8.0 to about 4.4, from about 8.0 to about 4.6, from about 8.0 to about 4.8, from about 8.0 to about 5.0, from about 8.0 to about 5.2, from about 8.0 to about 5.4, from about 8.0 to about 5.6, from about 8.0 to about 5.8, from about 8.0 to about 6.0, from about 7.8 to about 4.0, from about 7.8 to about 4.2, from about 7.8 to about 4.4, from about 7.8 to about 4.6, from about 7.8 to about 4.8, from about 7.8 to about 5.0, from about 7.8 to about 5.2, from about 7.8 to about 5.4, from about 7.8 to about 5.6, from about 7.8 to about 5.8, from about 7.6 to about 4.0, from about 7.6 to about 4.2, from about 7.6 to about 4.4, from about 7.6 to about 4.6, from about 7.6 to 4.8, from about 7.6 to about 5.0, from about 7.6 to about 5.2, from about 7.6 to about 5.4, from about 7.6 to about 5.6, from about 7.4 to about 4.0, from about 7.4 to about 4.2, from about 7.4 to about 4.4, from about 7.4 to about 4.6, from about 7.4 to about 4.8, from about 7.4 to about 5.0, from about 7.4 to about 5.2, from about 7.4 to about 5.4, from about 7.2 to about 4.0, from about 7.2 to about 4.2, from about 7.2 to about 4.4, from about 7.2 to about 4.6, from about 7.2 to about 4.8, from about 7.2 to about 5.0, from about 7.2 to about 5.2, from about 7.0 to about 4.0, from about 7.0 to about 4.2, from about 7.0 to about 4.4, from about 7.0 to about 4.6, from about 7.0 to about 4.8 and from about 7.0 to about 5.0.

Herein, depending on the individual separation conditions, the buffer A and/or buffer B can be prepared from a combination of a plurality of good buffers selected from the group consisting of, for example, MES, Bis-Tris, ADA, PIPES, ACES, MOPSO, BES, MOPS, TES, HEPES, TAPSO, POPSO, HEPSO, EPPS, Tricine, Bicine, TAPS, CHES, CAPSO and CAPS, respectively. In particular, when the solution containing the anti-PD-1/CD3 dual specificity antibody pertaining to the present invention is subjected to the separation method, it is preferable to prepare both of the buffers from a combination of good buffers selected from the group consisting of, for example, MES, MOPS, TAPS and CAPSO, in particular, preferably Thermo Scientific (registered trademark) CX-1 pH Gradient Buffer A (pH 5.6) and CX-1 pH Gradient Buffer B (pH 10.2) from ThermoFisher.

Furthermore, both of the buffers can also be prepared from a combination of a plurality of buffers selected from the group consisting of, for example, Tris-acetate, Tris-phosphate and Tris-citrate.

Depending on the nature of the protein to be subjected to the separation method, the conditions for the pH gradient can be predetermined so that the non-colorants and colorants can be separated. Herein, that "the non-colored protein and colored protein are separated" means that both proteins are separated under conditions predetermined so that the fraction containing the non-colored proteins recovered by the separation method or concentrated solution thereof is at a color concentration of "colorless or slightly colored", that the fraction containing the non-colored proteins recovered does not substantially contain the colorants, or that the remaining proportion of the colorants is reduced so as to be at a color concentration of "colorless or slightly colored" compared to the non-colorants.

In the present invention, the color concentration of "colorless or slightly colored" which is used as a definition of the degree of coloration, can be evaluated by qualitative or quantitative analysis. For example, the qualitative analysis can be performed by the visual comparison with a reference standard solution listed in the US Pharmacopoeia 2012 (USP Monograph 631, Color and Achromicity), the European Pharmacopoeia 5.0 (EP Method 2.2.2, Degree of Coloration of Liquids) or item 2.65 "Color comparison test" in the general tests of the Japanese Pharmacopoeia, 17th Edition. For example, if the coloration of protein in the present invention is substantially the same as or lighter than that of BY7 in the brownish-yellow reference standard solutions BY1 to BY7, it can be evaluated as "colorless", and if that is darker than that of BY7 but substantially the same as or lighter than that of BY5, it can be evaluated as "slightly colored" or "slightly yellow". Specifically, the Clarity, Opalescence and Coloration (COC) assay is performed by pouring the reference standard solution and the solution to be tested into a colorless, transparent and neutral glass test tube with flat bottom and 15-25 mm inner diameter so that the depth of layer is 40 mm, and comparing both.

Examples of the quantitative analysis include an evaluation method for calculating the Em/UV^{∗}100 value described above, which can be performed by measuring the UV absorption using a spectrophotometer and the fluorescence spectrum using a fluoro spectrophotometer. Herein, the brownish-yellow reference solution BY6 in the "color comparison test" corresponds to about 4.1 and BY5 corresponds to about 6.3, as the Em/UV^{∗}100 value.

It is preferable that the color concentration of the non-colored protein fraction or concentrated solution thereof, separated or fractionated by the present invention is evaluated to be substantially the same as or lighter than that of BY5 in the visual comparison test with the reference standard solution BY5, even more preferable that it is evaluated to be substantially the same as or lighter than that of BY6, and most preferable that it is evaluated to be substantially the same as that of BY7. Herein, it is more preferable that the concentrated solution of the non-colored protein fraction (wherein the protein concentration is greater than or equal to an arbitrary concentration in the range of from about 50 to about 100 mg/mL, preferably greater than or equal to a concentration selected from the group consisting of about 50 mg/mL, about 60 mg/mL, about 70 mg/mL, about 80 mg/mL, about 90 mg/mL and about 100 mg/mL) is evaluated to be substantially the same as or lighter than that of BY5, or even substantially the same as or lighter than that of BY6, or substantially the same as that of BY7. The concentration of the non-colored protein fraction can be carried out by well-known methods, such as ultrafiltration, precipitation and redissolution, and lyophilization and redissolution.

As an embodiment of the step-wise gradient among the pH gradients in the separation method of the present invention, the step-wise pH gradient from the initial pH to the terminal pH can be carried out by, for example, increasing the percentage (%) of the buffer B in the elution buffer by an arbitrary percentage in the range of from about 0.5 to about 5%, for example, any percentage selected from the group consisting of about 0.5%, about 1%, about 1.5%, about 2%, about 2.5%, about 3%, about 3.5%, about 4%, about 4.5% and about 5%, at each interval corresponding to an arbitrary column volume (CV (Column Volume) in the range of from about 1 to 20 CV, for example, any column volume selected from the group consisting of about 1 CV, about 2 CV, about 3 CV, about 4 CV, about 5 CV, about 6 CV, about 7 CV, about 8 CV, about 9 CV, about 10 CV, about 15 CV and about 20 CV. Herein, it can be performed by step by step increasing the percentage (%) of the buffer B, at least from any percentage in the range of from about 10 to about 100%, for example, any percentage selected from the group consisting of about 10%, about 20%, about 30%, about 40%, about 50%, about 60%, about 70%, about 70%, about 90% and about 100%, to any percentage in the range of from about 30 to about 100%, for example, any of the percentage selected from the group consisting of about 30%, about 40%, about 50%, about 60%, about 70%, about 80%, about 90% and about 100%. Specifically, the percentage (%) of the buffer B can be increased in the respective ranges of from about 10 to about 30%, from about 10 to about 40%, from about 10 to about 50%, from about 10 to about 60%, from about 10 to about 70%, from about 10 to about 80%, from about 10 to about 90%, from about 10 to about 100%, from about 20 to about 40%, from about 20 to about 50%, from about 20 to about 60%, from about 20 to about 70%, from about 20 to about 80%, from about 20 to about 90%, from about 20 to about 100%, from about 30 to about 50%, from about 30 to about 60%, from about 30 to about 70%, from about 30 to about 80%, from about 30 to about 90%, from about 30 to about 100%, from about 40 to about 60%, from about 40 to about 70%, from about 40 to about 80%, from about 40 to about 90%, from about 40 to about 100%, from about 50 to about 70%, from about 50 to about 80%, from about 50 to about 90%, from about 50 to about 100%, from about 60 to about 80%, from about 60 to about 90%, from about 60 to about 100%, from about 70 to about 90%, from about 70 to about 100% and from about 80 to about 100%. The "column volume (CV)" means the volume of gap between packing materials packed in a column, which means the amount of solvent required to fill gap, of which the volume is represented as "1CV".

Furthermore, as another embodiment of the step-wise gradient among the pH gradients in the separation method of the present invention, the step-wise pH gradient from the initial pH to the terminal pH can be performed by decreasing the pH of the elution buffer by an arbitrary value in the range of from about 0.1 to about 1.0, for example, any value selected from the group consisting of about 0.1, about 0.2, about 0.3, about 0.4, about 0.5, about 0.6, about 0.7, about 0.8, about 0.9 and about 1.0, at each interval corresponding to an arbitrary column volume in the range of from about 1 to about 20 CV, for example, any column volume selected from the group consisting of about 1 CV, about 2 CV, about 3 CV, about 4 CV, about 5 CV, about 6 CV, about 7 CV, about 8 CV, about 9 CV, about 10 CV, about 15 CV and about 20 CV

As yet another embodiment of the step-wise gradient among the pH gradients in the separation method of the present invention, the step-wise pH gradient from the initial pH to the terminal pH can be performed, by increasing the percentage (%) of the buffer B in the elution buffer within a range corresponding to an arbitrary column volume in the range of from about 20 to about 50 CV, for example, any column volume selected from the group consisting of about 20 CV, 25 CV, 30 CV, 35 CV, 40 CV, 45 CV and 50 CV, by an arbitrary percentage in the range of from about 0.5 to about 5%, for example, any percentage selected from the group consisting of about 0.5%, about 1%, about 1.5%, about 2%, about 2.5%, about 3%, about 3.5%, about 4%, about 4.5% and about 5%. Herein, it can be performed by step by step increasing the percentage (%) of the buffer B, at least from an arbitrary percentage in the range of from about 10 to about 100%, for example, any percentage selected from the group consisting of about 10%, about 20%, about 30%, about 40%, about 50%, about 60%, about 70%, about 80%, about 90% and about 100%, to an arbitrary percentage in the range of from about 30 to about 100%, for example, any percentage selected from the group consisting of about 30%, about 40%, about 50%, about 60%, about 70%, about 80%, about 90% and about 100%.

Similarly, as yet another form of the step-wise gradient among the pH gradients in the separation method of the present invention, the step-wise pH gradient from the initial pH to the terminal pH can be performed by decreasing the pH of the elution buffer by an arbitrary value in the range of from about 0.1 to about 1.0, for example, any value selected from the group consisting of about 0.1, about 0.2, about 0.3, about 0.4, about 0.5, about 0.6, about 0.7, about 0.8, about 0.9 and about 1.0, in a range corresponding to an arbitrary column volume in the range of from about 20 to about 50 CV, for example, any column volume selected from the group consisting of about 20 CV, about 25 CV, about 30 CV, about 35 CV, about 40 CV, about 45 CV and about 50 CV.

On the other hand, as an embodiment of the linear gradient among the pH gradients in the separation method of the present invention, the continuous pH gradient from the initial pH to the terminal pH can be performed by continuously increasing the percentage (%) of the buffer B in the elution buffer in a range corresponding to an arbitrary column volume in the range of from about 20 to about 50 CV, for example, any column volume selected from the group consisting of about 20 CV, 25 CV, 30 CV, 35 CV, 40 CV, 45 CV and 50 CV. Herein, this can be performed by continuously increasing the percentage (%) of the buffer B from at least an arbitrary percentage in the range of from about 10 to about 100%, for example, any percentage selected from the group consisting of about 10%, about 20%, about 30%, about 40%, about 50%, about 60%, about 70%, about 80%, about 90% and about 100%, to an arbitrary percentage in the range of from about 30% to about 100%, for example, any percentage selected from the group consisting of about 30%, about 40%, about 50%, about 60%, about 70%, about 80%, about 90% and about 100%.

As another embodiment of the linear pH gradient among the pH gradients in the separation method of the present invention, the continuous pH gradient from the initial pH to the terminal pH can be performed by continuously decreasing the pH of the elution buffer in a range corresponding to an arbitrary column volume in the range of from about 20 to about 50 CV, for example, any column volume selected from the group consisting of about 20 CV, 25 CV, 30 CV, about 35 CV, about 40 CV, about 45 CV and about 50 CV.

The pH gradient in the separation method of the present invention may be performed, for example, preferably, (a) by step-wise increasing the percentage (%) of the buffer B in the elution buffer, by an arbitrary percentage in the range of from about 1 to about 2%, at an interval corresponding to an arbitrary column volume in the range of from about 4 to about 6 CV, over the range of at least from about 50 to 100%, or (b) by step-wise decreasing the pH of the elution buffer, by an arbitrary pH in the range of from about 0.6 to about 1.0, over the range corresponding to an arbitrary column volume in the range of from about 30 to about 40 CV.

The elution buffer in the separation method of the present invention can be flowed at the optimum linear flow rate predetermined, according to the type of ion-exchange carrier used and the degree of separation between the colorants and non-colorants, which can be selected from an arbitrary linear flow rate in the range of from about 100 to about 800 cm/h, for example, the group consisting of about 100 cm/h, about 150 cm/h, about 200 cm/h, about 250 cm/h, about 300 cm/h, about 350 cm/h, about 400 cm/h, about 450 cm/h, about 500 cm/h, about 550 cm/h, about 600 cm/h about 650 cm/h, about 700 cm/h, about 750 cm/h and about 800 cm/h. Herein, the linear flow rate is the rate at which the elution buffer passes through the column crosssection of the ion-exchange carrier column, and is calculated by the following equation: Linear flow rate (cm/h) = Flow rate (cm³/h) / Column cross-sectional area (cm²).

In the separation method of the present invention, the total loadings (mg/mL) of non-colored proteins and colored proteins which are loaded into the ion-exchange carrier column are from about 2 to about 200 mg/mL, examples of which include about 10 mg/mL, about 20 mg/mL, about 30 mg/mL, about 40 mg/mL, about 50 mg/mL, about 60 mg/mL, about 70 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, and about 200 mg/mL. Herein, the loading volume (mg/mL) refers to the amount of sample added per volume of an ion-exchange carrier column.

The total concentrations of the non-colored proteins and colored proteins which can be loaded into an ion-exchange carrier column depend on the conditions described above as well, but which are usually from about 2 to about 50 mg/mL, specifically, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL and about 50 mg/mL, and preferably from about 10 to about 30 mg/mL.

The separation method of the present invention is mainly performed at room temperature.

The term "about" as used herein means that the value may vary under or over the indicated value within the range of 10%.

### [Protein in the present invention]

The separation method of the present invention can be applied to any proteins colored by modifications due to glycosylation reactions. In particular, protein drugs which are required to be at high concentration are susceptible to coloring due to glycosylation reactions because they need to be produced from cultured cells (e.g., COS cells or CHO cells) under high oxygen and glucose conditions. Antibodies are particularly required to be applied to this separation method.

Basically, the separation method of the present invention can be broadly applied to proteins having any molecular weight, but specifically to those which fall within the range of from about 50 to 750 kDa, and is suitable for separation of proteins which fall within the range of from about 50 to about 150 kDa.

Furthermore, basically, the separation method of the present invention can be widely applied to proteins having any isoelectric point, but preferably those which fall within the range of from about 5 to 9.5 are targeted. Among colored proteins, those of which the isoelectric points fall within the range of from about 5 to about 9.1, but mainly within the range of from about 6 to about 7 are targeted. On the other hand, among non-colored proteins, those of which the isoelectric points fall within the range of from about 7 to about 9.5, specifically, within any range selected form the group consisting of about 7 or more but less than about 7.5, about 7.5 or more but less than about 8, about 8 or more but less than about 8.5, about 8.5 or more but less than about 9, and about 9 or more but less than about 9.5 are targeted.

The antibodies to which the separation method of the present invention can be applied may be of any kind or form such as monoclonal antibodies, polyclonal antibodies, chimeric antibodies, humanized antibodies, human antibodies and multi-specific antibodies (e.g., bispecific antibodies). The species of such antibodies is not limited, for example, mouse, rat, rabbit, goat or human origin antibodies may be used, but for drugs for human of which the quality is strictly required, those of chimeric antibodies, humanized antibodies, or fully humanized antibodies are preferable. In addition, since they are mostly generated as monoclonal antibodies from producing cells such as COS or CHO cells, the coloring due to glycation reactions may become a problem.

In the present invention, the term "antibody fragment" means a portion of antibody molecule which retains its binding to an antigen, and includes, for example, F(ab')₂, Fab', Fab, Fv and single-chain FV.

In the present specification, the term "bispecific antibody" means an antibody having the binding specificity to two different antigen molecules or epitopes on one molecule. Herein, examples of forms of the bispecific antibodies include a diabody, bispecific sc(Fv)₂, bispecific minibody, bispecific F(ab')₂, bispecific hybrid antibody, covalent diabody (bispecific DART), bispecific (FvCys)₂, bispecific F(ab'-zipper)₂, bispecific (Fv-zipper)₂, bispecific three-chain antibody and bispecific mAb² and the like.

In the present specification, the term "isotype" means the antibody class (e.g., IgM or IgG) which is encoded by heavy chain constant region genes. The preferable isotype for the bispecific antibody of the present invention is IgG, more preferably IgGi or IgG₄.

Examples of the antibodies to which the separation method of the present invention can be applied include anti-CD40 antibody (e.g., Bleselumab, Dacetuzumab, Iscalimab, Lucatumumab, Mitazalimab, Ravagalimab, Selicrelumab, Teneliximab, ABBV-428 and APX005M, etc.), anti-CD70 antibody (e.g., Cusatuzumab, Vorsetuzumab andARGX-110, etc.), anti-HER1 antibody (e.g., Cetuximab, Panitumumab, Necitumumab, Nimotuzumab, Depatuxizumab, Futuximab, Laprituximab, Matuzumab, Modotuximab, Petosemtamab, Tomuzotuximab Losatuxizumab, Serclutamab, Imgatuzumab, Futuximab and Zalutumumab, etc.), anti-HER2 antibody (e.g., Pertuzumab, Disitamab, Gancotamab, Margetuximab, Timigutuzumab, Zanidatamab, Ertumaxomab, Zenocutuzumab, Trastuzumab, MM-111, R48 and ZW33, etc.), anti-HER3 antibody (e.g., Duligotuzumab, Elgemtumab, Istiratumab, Lumretuzumab, Zenocutuzumab, Patritumab, Seribantumab and MM-111, etc.), anti-VEGFR1 antibody (e.g., Icrucumab etc.), anti-VEGFR2 antibody (e.g., Ramucirumab, Alacizumab and Olinvacimab, etc.), anti-CD20 antibody (e.g., Rituximab, Blontuvetmab, Epitumomab, Ibritumomab, Ocaratuzumab, Ocrelizumab, Nofetumomab, Tositumomab, Veltuzumab, Ofatumumab, Ublituximab, Obinutuzumab and Nofetumomab, etc.), anti-CD30 antibody (e.g., Brentuximab and Iratumumab, etc.), anti-CD38 antibody (e.g., Daratumumab, Isatuximab, Mezagitamab, AT13/5 and MOR202, etc.), anti-TNFRSF10B antibody (e.g., Benufutamab, Conatumumab, Drozitumab, Lexatumumab, Tigatuzumab and DS-8273a, etc.), anti-TNFRSF10A antibody (e.g., Mapatumumab etc.), anti-MUC1 antibody (e.g., Cantuzumab, Clivatuzumab, Epitumomab, Sontuzumab, Gatipotuzumab, Nacolomab, 7F11C7, BrE-3, CMB-401, CTM01 and HMFG1, etc.), anti-MUC5AC antibody (e.g., Ensituximab etc.), anti-MUC16 antibody (e.g., Oregovomab, Abagovonnab, Igovomab and Sofituzumab, etc.), anti-DLL4 antibody (e.g., Demcizumab, Dilpacimab, Navicixizumab and Enoticumab, etc.), antifucosyl GM1 antibody (e.g., BMS-986012 etc.), anti-gpNMB antibody (e.g., Glembatumumab etc.), anti-Mesothelin antibody (e.g., Amatuximab, Anetumab, RG7784 and BMS-986148, etc.), anti-MMP9 antibody (e.g., Andecaliximab etc.), anti-GD2 antibody (e.g., Dinutuximab, Naxitamab, 14G2a, MORAb-028, Surek, TRBs07 and ME361, etc.), anti-MET antibody (e.g., Emibetuzumab, Onartuzumab and Telisotuzumab, etc.), anti-FOLR1 antibody (e.g., Farletuzumab and Mirvetuximab, etc.), anti-CD79b antibody (e.g., Iladatuzumab and Polatuzumab, etc.), anti-DLL3 antibody (e.g., Rovalpituzumab etc.), anti-CD51 antibody (e.g., Abituzumab, Etaracizumab and Intetumumab, etc.), anti-EPCAM antibody (e.g.Adecatumumab, Catumaxomab, Edrecolomab, Oportuzumab, Citatuzumab and Tucotuzumab, etc.), anti-CEACAM5 antibody (e.g., Altumomab, Arcitumomab, Cergutuzumab, Labetuzumab, 90Y-cT84.66, AMG211, BW431/26, CE25/B7, COL-1 and T84.66 M5A, etc.), anti-CEACAM6 antibody (e.g., Tinurilimab etc.), antibodyFGFR2 antibody (e.g., Aprutumab and Bemarituzumab, etc.), anti-CD44 antibody (e.g., Bivatuzumab etc.), anti-PSMA antibody (e.g., Capromab, 177Lu-J591 and ES414, etc.), anti-Endoglin antibody (e.g., Carotuximab etc.), anti-IGF1R antibody (e.g., Cixutumumab, Figitumumab, Ganitumab, Dalotuzumab, Teprotumumab and Robatumumab, etc.), anti-TNFSF11 antibody (e.g., Denosumab etc.), anti-GUCY2C (e.g., Indusatumab etc.), anti-SLC39A6 antibody (e.g., Ladiratuzumab etc.), anti-SLC34A2 antibody (e.g., Lifastuzumab etc.), anti-NCAM1 antibody (e.g., Lorvotuzumab and N901, etc.), anti-ganglioside GD3 antibody (e.g., Ecromeximab and Mitumomab, etc.), anti-AMHR2 antibody (e.g., Murlentamab etc.), anti-CD37 antibody (e.g., Lilotomab, Naratuximab and Otlertuzumab, etc.), anti-IL1RAP antibody (e.g., Nidanilimab etc.), anti-PDGFR2 antibody (e.g., Olaratumab and Tovetumab, etc.), anti-CD200 antibody (e.g., Samalizumab etc.), anti-TAG-72 antibody (e.g., Anatumomab, Minretumomab, Satumomab, CC49, HCC49 and M4, etc.), anti-SLITRK6 antibody (e.g., Sirtratumab etc.), anti-DPEP3 antibody (e.g., Tamrintamab etc.), anti-CD19 antibody (e.g., Coltuximab, Denintuzumab, Inebilizumab, Loncastuximab, Obexelimab, Tafasitamab, Taplitumomab and huAnti-B4, etc.), anti-NOTCH2/3 antibody (e.g., Tarextumab etc.), anti-tenascin C antibody (e.g., Tenatumomab etc.), anti-AXL antibody (e.g., Enapotamab and Tilvestamab, etc.), anti-STEAP1 antibody (e.g., Vandortuzumab etc.), anti-CTAA16 antibody (e.g., Votumumab etc.), CLDN18 antibody (e.g., Zolbetuximab etc.), anti-GM3 antibody (e.g., Racotumomab, FCGR1 and H22, etc.), anti-PSCA antibody (e.g., MK-4721 etc.), anti-FN extra domain B antibody (e.g., AS1409 etc.), anti-HAVCR1 antibody (e.g., CDX-014 etc.), anti-TNFRSF4 antibody (e.g., MEDI6383 etc.), anti-PD-1 antibody (e.g., Nivolumab, Cemiplimab-rwlc, Pembrolizumab, Spartalizumab, Tislelizumab, Dostarlimab, Toripalimab, Camrelizumab, Genolimzumab, Sintilimab, Lodapolimab, Retifanlimab, Balstilimab, Serplulimab, Budigalimab, Prolgolimab, Sasanlimab, Cetrelimab, Zimberelimab and Geptanolimab, etc.), anti-PD-L1 antibody (e.g., Atezolizumab, Avelumab, Durvalumab, Manelimab, Pacmilimab, Envafolimab, Cosibelimab and Sugemalimab, etc.), anti-CTLA-4 antibody (e.g., Ipilimumab, Zalifrelimab, Nurulimab and Tremelimumab, etc.), anti-TIM3 antibody (e.g., Cobolimab and MBG453, etc.), anti-CD3 antibody (e.g., Catumaxomab etc.), anti-CD4 antibody (e.g., Zanolimumab and IT1208, etc.), anti-CD27 antibody (e.g., Varlilumab etc.), anti-CD73 antibody (e.g., Oleclumab and BMS-986179, etc.), anti-CD80 antibody (e.g., Galiximab etc.), anti-CD86 antibody, anti-CD160 antibody, anti-CD57 antibody, anti-CD226 antibody, anti-CD112 antibody, anti-CD155 antibody, anti-OX40 antibody (e.g., MEDI6469, Ivuxolimab, MEDI0562, MEDI6383, Efizonerinnod, GSK3174998, BMS-986178 and MOXR0916, etc.), OX40L antibody (e.g., Oxelumab and Tavolimab, etc.), anti-ICOS antibody (e.g., Vopratelimab and GSK3359609, etc.), anti-4-1BB (CD137) antibody (e.g., Urelumab and Utomilumab, etc.), anti-4-1BBL (CD137L) antibody, anti-2B4 antibody, anti-GITR antibody (e.g., MK-4166, INCAGN01876, GWN323 and TRX-518, etc.), anti-B7-H3 antibody (e.g., Enoblituzumab, Mirzotamab and Omburtamab, etc.), anti-LAG-3 antibody (e.g., Relatlimab, Ieramilimab, Fianlimab, Encelimab and Mavezelimab, etc.), anti-BTLA antibody, anti-HVEM antibody, anti-VISTA antibody (e.g., Onvatilimab etc.), anti-GITRL antibody, anti-Galectin-9 antibody, anti-B7-H4 antibody, anti-B7-H5 antibody, anti-PD-L2 antibody, anti-KLRG-1 antibody, anti-E-Cadherin antibody, anti-N-Cadherin antibody, anti-R-Cadherin antibody, anti-CSF-1R antibody (e.g., Cabiralizumab, Emactuzumab, LY3022855, Axatilimab, MCS-110, IMC-CS4, AMG820, Pexidartinib, BLZ945 and ARRY-382, etc.), anti-CXCR4 antibody (e.g., Ulocuplumab etc.), anti-FLT-3 antibody, anti-TIGIT antibody (e.g., Tiragolumab, Etigilimab, Vibostolimab and BMS-986207, etc.), anti-KIR antibody (e.g., Lirilumab, IPH2101, LY3321367 and MK-4280, etc.), anti-SLAMF7 antibody (e.g., Azintuxizumab and Elotuzumab, etc.), anti-CD47 antibody and anti-NKG2A antibody (e.g., Monalizumab etc.) and the like.

Examples of the bispecific antibodies to which the separation method of the present invention can be applied include anti-PD-1/CD3 bispecific antibody, anti-PD-1/CD19 bispecific antibody (preferably, the bispecific antibodies described in WO2020/204152), anti-PD-1/CD4 bispecific antibody, anti-HER1-MET bispecific antibody (e.g., Amivantamab etc.), anti-EPCAM-CD3 bispecific antibody (e.g., Solitomab and Catumaxomab, etc.), anti-Ang2-VEGF bispecific antibody (e.g., Vanucizumab etc.), anti-HER2-CD3 bispecific antibody (e.g., Ertumaxomab etc.), anti-HER3-IGF1R bispecific antibody (e.g., Istiratumab etc.), anti-PMSA-CD3 bispecific antibody (e.g., Pasotuxizumab etc.), anti-HER1-LGR5 bispecific antibody (e.g., Petosemtamab etc.), anti-SSTR2-CD3 bispecific antibody (e.g., Tidutamab etc.), anti-CD30-CD16A bispecific antibody (e.g., AFM13 etc.), anti-CEA-CD3 bispecific antibody (e.g., Cibisatamab and RO6958688, etc.), anti-CD3-CD19 bispecific antibody (e.g., Duvortuxizumab and Blinatumomab, etc.), anti-IL3RA-CD3 bispecific antibody (e.g., Flotetuzumab and Vibecotamab, etc.), anti-GPRC5D-CD3 bispecific antibody (e.g., Talquetamab etc.), anti-TNFRSF17-CD3 bispecific antibody (e.g., Teclistamab etc.), anti-HER2-HER3 bispecific antibody (e.g., Zenocutuzumab etc.) and anti-CD20-CD3 bispecific antibody (e.g., Plamotamab, Odronextamab, Mosunetuzumab, Epcoritamab, Glofitamab and REGN1979, etc.) and the like.

Examples of the antibodies to which the separation method of the present invention is applied include an anti-PD-1/CD3 bispecific antibody, particularly, a solution containing the bispecific antibody which comprises the heavy chain and light chain constituting the antigen-binding site specifically binding to PD-1 and the heavy chain and light chain constituting the antigen-binding site specifically binding to CD3, and wherein (a) the heavy chain among the heavy chain and light chain constituting the antigen-binding site specifically binding to PD-1 comprises the amino acid sequence set forth in any one selected from SEQ ID No. 1, SEQ ID No. 2, SEQ ID No. 3, SEQ ID No. 4 and SEQ ID No. 5 (preferably, the amino acid sequence of SEQ ID No. 5), (b) the heavy chain among the heavy chain and light chain constituting the antigen-binding site specifically binding to CD3 comprises the amino acid sequence set forth in SEQ ID No. 6, and (c) both of the light chain among the heavy chain and light chain constituting the antigen-binding site specifically binding to PD-1 and the light chain among the heavy chain and light chain constituting the antigen-binding site specifically binding to CD3 comprise the amino acid sequences set forth in SEQ ID No. 7 (the anti-PD-1/CD3 bispecific antibody A) is recognized to be colored due to the generation of AGEs, which requires the separation method of the present invention.

The anti-PD-1/CD3 bispecific antibody A can be produced according to the method disclosed in WO2019/156199, which corresponds to the anti-PD-1/CD3 bispecific antibody clones PD1-1(Bi) to PD1-5(Bi), respectively.

Furthermore, anti-PD-1/CD3 bispecific antibodies (anti-PD-1/CD3 bispecific antibody B) of which the heavy chain constant region of the heavy chain among the heavy chain and light chain constituting the antigen-binding site specifically binding to PD-1 of the foregoing anti-PD-1/CD3 bispecific antibody A is substituted by the heavy chain constant region comprising the amino acid sequence set forth in any one selected from SEQ ID No. 8, SEQ ID No. 9, SEQ ID No. 10, SEQ ID No. 11, SEQ ID No. 12 and SEQ ID No. 13, and the heavy chain constant region of the heavy chain among the heavy chain and light chain constituting the antigen-binding site specifically binding to CD3 is substituted by the heavy chain constant region comprising the amino acid sequence set forth in any one selected from SEQ ID No. 14, SEQ ID No. 15, SEQ ID No. 16, SEQ ID No. 17, SEQ ID No. 18 and SEQ ID No. 19 require the separation method of the present invention, as well.

These anti-PD-1/CD3 bispecific antibody B correspond to the antibody clones in which methionine at position 252 according to the EU numbering system in the heavy chain constant region of the anti-PD-1/CD3 bispecific antibody clone PD1-5(Bi) (the anti-PD-1/CD3 bispecific antibody A of which the heavy chain among the heavy chain and light chain constituting the antigen-binding site specifically binding to PD-1 comprises the amino acid sequence set forth in SEQ ID No. 5) was substituted with glutamic acid, proline, arginine and aspartic acid, respectively, the antibody clone in which asparagine at position 434 was substituted with leucine, and the antibody clone in which glutamine at position 438 was substituted with glutamic acid, respectively, which can be prepared in accordance with well-known techniques on amino acid substitutions.

Furthermore, anti-PD-1/CD3 bispecific antibodies which cross-compete with the binding of the anti-PD-1/CD3 bispecific antibody A to PD-1 and/or CD3, respectively, may require the separation method of the present invention, as well. Herein, the term "crosscompeting" means inhibiting the binding of the anti-PD-1/CD3 bispecific antibody A to PD-1 and/or CD3, regardless of the degree thereof, by binding to the respective epitopes on PD-1 and/or CD3 which are the same as or partially overlap with those of the anti-PD-1/CD3 bispecific antibody A, and whether it cross-competes or not can be evaluated by competitive binding assays. For example, it can be determined using Biacore analysis, ELISA assay, flow cytometry, enzyme linked immunosorbent assay (ELISA), fluorescence energy transfer method (FRET) and fluorometric micro volume assay technology (FMAT (registered trademark)).

Figure 9 shows the respective amino acid sequences of the heavy chain and light chain constituting the antigen-binding site specifically binding to PD-1 and the heavy chain and light chain constituting the antigen-binding site specifically binding to CD3, constituting the anti-PD-1/CD3 bispecific antibody A. Figures 10 and 11 show the respective amino acid sequences of the heavy chain constant regions constituting the anti-PD-1/CD3 bispecific antibody B.

Herein, the "heavy chain and light chain constituting the antigen-binding site specifically binding to PD-1" means the heavy chain and light chain complexes forming the antigen-binding site specifically binding to PD-1 by association of the heavy chain and light chain through disulfide bonds. The "antigen-binding site" is the smallest unit for antibody to have the binding activity to its antigen. The term "specifically binding to PD-1" is used as a feature of directly binding to PD-1 with higher binding activity than at least 1 × 10⁻⁵ M, preferably 1 × 10⁻⁷ M, and more preferably 1 × 10⁻⁹ M affinity (dissociation constant (Kd value)), and not substantially binding to any receptor members belonging to a so-called CD28 family receptor, such as at least CD28, CTLA-4 and ICOS.

The "heavy chain and light chain constituting the antigen-binding site specifically binding to CD3" means the heavy chain and light chain complexes forming the antigen-binding site specifically binding to CD3 by association of the heavy chain and light chain through disulfide bonds. The "antigen-binding site" is the smallest unit for antibody to have the binding activity to its antigen. The term "specifically binding to CD3" is used as a feature of directly binding to CD3 with higher binding activity than at least 1 × 10⁻⁵ M, preferably 1 × 10⁻⁷ M, and more preferably 1 × 10⁻⁹ M affinity (dissociation constant (Kd value)), and not substantially binding to any other proteins.

The foregoing anti-PD-1/CD3 bispecific antibody of the present invention is characterized by the structure that the amino acid sequences of the light chain among the heavy chain and light chain constituting the antigen-binding site specifically binding to PD-1 and the light chain among the heavy chain and light chain constituting the antigen-binding site specifically binding to CD3 are the same.

A preferable embodiment of the separation method of the present invention is, for example, a method for separating non-colored proteins from a solution containing non-colored proteins and colored proteins,
(1) wherein the separation method comprises
   (i) a step for loading the solution onto an anion-exchange carrier column, and using an equilibration buffer at a predetermined initial pH, making the non-colored proteins and colored proteins bind to anion-exchange carriers thereof,
   (ii) a step for flowing an elution buffer, forming a pH gradient which descends from the initial pH to the terminal pH, through the anion-exchange carrier column at a predetermined flow rate, under a condition predetermined to separate the non-colored proteins and colored proteins; and
   (iii) a step for collecting a predetermined fraction containing the non-colored proteins eluted by the preceding step (ii),
(2) wherein the protein is any of the anti-PD-1/CD3 bispecific antibody A or any of the anti-PD-1/CD3 bispecific antibody B,
(3) wherein the anion-exchange carrier is Toyopearl (registered trademark) NH2-750F,
(4) wherein the initial pH of the equilibration buffer and elution buffer is an arbitrary pH in the range of from about 10.6 to about 8.4, and the terminal pH of the elution buffer is an arbitrary pH in the range of from about 7.0 to about 4.0 (provided that the initial pH is at least 2.0 higher than the terminal pH),
(5) wherein the pH gradient is performed (a) by increasing the percentage (%) of the buffer B in the elution buffer step-by-step by from about 1 to about 2 %, at an interval corresponding to an arbitrary column volume in the range of from about 4 to about 6 CV, over at least the range of from about 50 to 100%, or (b) by decreasing the pH of the elution buffer step-by-step by an arbitrary pH in the range of from about 0.6 to about 1.0, in a range corresponding to an arbitrary column volume in the range of from about 30 to about 40 CV,
(6) wherein the linear flow rate of the elution buffer is from about 100 to about 800 cm/h, and
(7) wherein the color concentration of the collected solution containing the non-colored proteins is (i) substantially the same as or lighter than that of BY5 in the visual comparison test with the reference standard solution BY5, or (ii) the Em/UV*100 value of the collected solution containing the non-colored proteins is about 6.3 or less.

Another preferable embodiment of the separation method of the present invention is, for example, a method for separating non-colored proteins from a solution containing non-colored proteins and colored proteins,
(1) wherein the separation method comprises
   (i) a step for loading the solution onto an anion-exchange carrier column,
   (ii) a step for flowing an equilibration buffer prepared so that the colored proteins in the solution bind to anion-exchange carriers thereof and the non-colored proteins do not, through the anion-exchange carrier column at a predetermined flow rate, and
   (iii) a step for collecting the fraction containing the non-colored proteins flowed through in the preceding step (ii) as it is,
(2) wherein the protein is any of the anti-PD-1/CD3 bispecific antibody A or any of the anti-PD-1/CD3 bispecific antibody B,
(3) wherein the anion-exchange carrier is Toyopearl (registered trademark) NH2-750F,
(4) wherein the pH of the equilibration buffer is an arbitrary pH in the range of from about 7.8 to about 8.2, and the electrical conductivity is an arbitrary electrical conductivity in the range of from about 5 to about 7 mS/cm,
(5) wherein the linear flow rate of the equilibration buffer is from about 100 to about 400 cm/h, and
(6) wherein the color concentration of the collected solution containing the non-colored proteins is (i) substantially the same as or lighter than that of BY5 in the visual comparison test with the reference standard solution BY5, or (ii) the Em/UV*100 value of the collected solution containing the non-colored proteins is about 6.3 or less.

### [A solution containing the anti-PD-1/CD3 bispecific antibody pertaining to the present invention]

The present invention relates to a solution containing an anti-PD-1/CD3 bispecific antibody produced by a production method including, as a part thereof, a process corresponding to the separation method of the present invention. Specifically, it is a colorless or slightly colored solution containing any of anti-PD-1/CD3 bispecific antibody A or any of anti-PD-1/CD3 bispecific antibody B at a concentration of from about 100 to about 300 mg/mL. However, preferable embodiments of the solutions containing the bispecific antibody do not include a solution having colorless or slightly colored properties at a concentration of from about 100 to about 300 mg/mL, without any process for reducing or removing the coloration, such as the separation method of the present invention, in any of producing processes thereof.

The solution containing any of anti-PD-1/CD3 bispecific antibody A or any of anti-PD-1/CD3 bispecific antibody B may be produced by subjecting it to the separation method of the present invention at a concentration of less than about 100 mg/mL, for example, any concentration from about 10 to 50 mg/mL, and then concentrating as necessary. The concentration of a non-colored protein fraction can be carried out by well-known methods, such as ultrafiltration, precipitation-redissolution, and lyophilization-redissolution. The color concentration of the colorless or slightly colored solution can be confirmed by qualitative or quantitative analysis as described above.

The solution containing an anti-PD-1/CD3 bispecific antibody produced by the production method including, as a part thereof, the process corresponding to the separation method of the present invention is useful for preventing, suppressing the progression of symptoms of, suppressing the recurrence of, and/or treating autoimmune diseases or graft-versus-host diseases (GVHD) or hematological cancer.

Examples of autoimmune diseases which can be prevented, of which the progression of symptoms can be suppressed and/or which can be treated with the PD-1/CD19 bispecific antibody pertaining to the present invention include Behcet's disease, systemic lupus erythematosus, chronic discoid lupus erythematosus, multiple sclerosis (systemic scleroderma and progressive systemic sclerosis), scleroderma, polymyositis, dermatomyositis, periarteritis nodosa (polyarteritis nodosa and microscopic polyangiitis), aortitis syndrome (Takayasu's arteritis), malignant rheumatoid arthritis, rheumatoid arthritis, juvenile idiopathic arthritis, spondyloarthritis, mixed connective tissue disease, Sjogren's syndrome, adult Still's disease, vasculitis, allergic granulomatous vasculitis, hypersensitivity vasculitis, rheumatoid vasculitis, large vessel vasculitis, ANCA associated vasculitis (e.g., granulomatosis with polyangiitis and eosinophilic granulomatosis with polyangiitis), Cogan's syndrome, RS3PE syndrome, temporal arteritis, polymyalgia rheumatica, fibromyalgia, antiphospholipid antibody syndrome, eosinophilic fasciitis, IgG₄-related disease (e.g., primary sclerosing cholangitis and autoimmune insulitis, etc.), Guillain-Barre syndrome, myasthenia gravis, chronic atrophic gastritis, autoimmune hepatitis, non-alcoholic steatohepatitis, primary biliary cirrhosis, Goodpasture's syndrome, rapidly progressive glomerulonephritis, megaloblastic anemia, autoimmune hemolytic anemia, pernicious anemia, autoimmune neutropenia, idiopathic thrombocytopenic purpura, Basedow disease (Graves' disease (hyperthyroidism)), Hashimoto disease, autoimmune adrenal insufficiency, primary hypothyroidism, Addison's disease (chronic hypoadrenocorticism), idiopathic Addison's disease, type I diabetes mellitus, slowly progressive type I diabetes mellitus (latent autoimmune diabetes in adult), focal scleroderma, psoriasis, psoriatic arthritis, bullous pemphigoid, pemphigus, pemphigoid, gestational herpes, linear IgA bullous dermatosis, acquired epidermolysis bullosa, alopecia areata, vitiligo, vitiligo vulgaris, neuromyelitis optica, chronic inflammatory demyelinating polyneuropathy, multifocal motor neuropathy, sarcoidosis, giant cell arteritis, amyotrophic lateral sclerosis, Harada disease, autoimmune optic neuropathy, idiopathic azoospermia, habitual abortion, inflammatory bowel disease (e.g., ulcerous colitis and Crohn's disease), celiac disease, ankylosing spondylitis, severe asthma, chronic urticaria, transplantation immunity, familial mediterranean fever, eosinophilic chronic rhinosinusitis, dilated cardiomyopathy, systemic mastocytosis, inclusion body myositis and the like.

Furthermore, examples of hematological cancer which can be prevented, of which the progression of symptoms or the recurrence can be suppressed, and/or which can be treated by the PD-1/CD3 bispecific antibody pertaining to the present invention include multiple myeloma, malignant lymphoma (e.g., non-Hodgkin's Lymphoma (e.g., B-cell non-Hodgkin's lymphoma (e.g., precursor B-cell lymphoblastic lymphoma, precursor B-cell acute lymphoblastic leukemia, chronic B-lymphoid leukemia (e.g., small lymphocytic lymphoma), B-cell precursor acute lymphoblastic leukemia, B-cell prolymphocytic leukemia, lymphoplasmacytic lymphoma, nodal marginal zone B-cell lymphoma, extranodal marginal zone B-cell lymphoma (MALT lymphoma), primary splenic marginal zone B-cell lymphoma, hairy cell leukemia, hairly cell leukemia-variant, follicular lymphoma, pediatric type follicular lymphoma, diffuse large B cell lymphoma, diffuse large B-cell lymphoma, not otherwise specified, splenic diffuse red pulp small B-cell lymphoma, lymphoplasmacytic lymphoma, primary mediastinal large B-cell lymphoma, primary effusion lymphoma, Burkitt's lymphoma, mantle cell lymphoma, monoclonal B-cell lymphocytosis, splenic B-cell lymphoma/leukemia, unclassifiable, IgM monoclonal gammopathy of undetermined significance, µ heavy chain disease, λ heavy chain disease, α heavy chain disease, plasma cell myeloma, solitary plasmacytoma of bone, extraosseous plasmacytoma, monoclonal immunoglobulin deposition disease, large B-cell lymphoma with IRF4 rearrangement, primary cutaneous follicle center lymphoma, T-cell/histiocyte-rich large B-cell lymphoma, primary diffuse large B-cell lymphoma of the central nervous system, primary cutaneous diffuse large B-cell lymphoma, leg type, EBV positive diffuse large B-cell lymphoma, not otherwise specified, EBV positive mucocutaneous ulcer, diffuse large B-cell lymphoma associated with chronic inflammation, lymphomatoid granulomatosis, intravascular large B-cell lymphoma, ALK positive large B-cell lymphoma, plasmablastic lymphoma, primary effusion lymphoma, HHV8 positive diffuse large B-cell lymphoma, not otherwise specified, Burkitt-like lymphoma with 11q aberration, high-grade B-cell lymphoma, with MYC and BCL2 and/or BCL6 rearrangement, high-grade B-cell lymphoma, not otherwise specified, or B-cell lymphoma, unclassifiable, with intermediate features between diffuse large B-cell lymphoma and classical Hodgkin lymphoma), T/NK-cell non-Hodgkin's lymphoma (e.g., precursor T-cell lymphoblastic lymphoma, chronic T-cell lymphocytic leukemia, T-cell large granular lymphoblastic lymphoma, NK-cell large granular leukemia, aggressive NK-cell leukemia, peripheral T-cell lymphoma, peripheral T-cell lymphoma, not otherwise specified, unclassifiable peripheral T-cell lymphoma, angioimmunoblastic T-cell lymphoma, (CD30-positive) anaplastic large cell lymphoma, angiocentric lymphoma, intestinal T-cell lymphoma, enteropathy-type T-cell lymphoma, hepatosplenic gamma-delta T cell lymphoma, subcutaneous panniculitis-like T-cell lymphoma, mycosis fungoides, Sezary syndrome, Hodgkin-like/Hodgkin-related anaplastic large cell lymphoma, extranodal NK/T-cell lymphoma, adult T cell leukemia/lymphoma, T-cell prolymphocytic leukemia, chronic lymphoproliferative disorder of NK-cells, systemic EBV positive T-cell lymphoma of childhood, hydroa vacciniforme-like lymphoproliferative disorder, extranodal NK/T-cell lymphoma, nasal type, enteropathy-associated T-cell lymphoma, monomorphic epitheliotropic intestinal T-cell lymphoma, indolent T-cell lymphoproliferative disorder of the GI tract, hepatosplenic T-cell lymphoma, primary cutaneous CD30 positive T-cell lymphoproliferative disorders, lymphomatoid papulosis, primary cutaneous anaplastic large cell lymphoma, primary cutaneous gamma-delta T-cell lymphoma, primary cutaneous CD8 positive aggressive epidermotropic cytotoxic T-cell lymphoma, primary cutaneous acral CD8 positive T-cell lymphoma, primary cutaneous CD4 positive small/medium T-cell lymphoproliferative disorder, follicular T-cell lymphoma, nodal peripheral T-cell lymphoma with follicular helper T cell phenotype, anaplastic large cell lymphoma, ALK positive, anaplastic large cell lymphoma, ALK negative, or breast implant-associated anaplastic largecell lymphoma)), and Hodgkin's Lymphoma (e.g., classical Hodgkin's lymphoma (e.g., nodular sclerosis, mixed cellularity, lymphocyte-rich and lymphopenic) or nodular lymphocyte predominant Hodgkin's lymphoma)), leukemia (e.g., acute myelogenous leukemia, acute promyelocytic leukemia, acute lymphoblastic leukemia (lymphoblastic lymphoma), chronic lymphocytic leukemia (small lymphocytic lymphoma), myelodysplastic syndrome, and chronic myelogenous leukemia), primary central nervous system lymphoma, myeloproliferative syndrome and the like.

In the present invention, the term "treating" means cure or improvement of certain disease or symptom thereof. The term "preventing" means that the onset of certain disease or symptom thereof is prevented or delayed for a certain period of time. The term "suppressing the progression of symptoms" means that the progress or aggravation of symptoms is suppressed to stop the progress of disease conditions. The meaning of "preventing" also includes suppressing the recurrence. The term "suppressing the recurrence" means that the recurrence of certain disease or syndrome thereof is prevented or a possibility of recurrence is reduced.

The solution containing the PD-1/CD3 bispecific antibody of the present invention is usually administered systemically or locally through parenteral administration. Specific examples of such administration methods include injection administration, intranasal administration, transpulmonary administration, percutaneous administration and the like. Examples of injection administration include intravenous injection, intramuscular injection, intraperitoneal injection and the like. For intravenous injection, drip intravenous infusion is preferable. The dose thereof varies depending on the age, body weight, symptoms, therapeutic effect, administration method, treating period and the like. The single dose thereof for an adult is usually in the range of 0.1 µg/kg to 300 mg/kg, particularly preferably, in the range of 0.1 mg/kg to 10 mg/kg, once to several times per day by parenteral administration, or in the range of 30 minutes to 24 hours per day by intravenous sustaining administration. Needless to say, as mentioned above, since the dose varies depending on various conditions, it may be lower than the above-mentioned dose, or may be needed to be more than the above.

### [Formulation]

When the solution containing the PD-1/CD3 bispecific antibody of the present invention is formulated to be used as an injection or infusion solution for drip infusion, the injection or infusion solution may be in any form of an aqueous solution, suspension or emulsion. Examples of solvents which can be used in the injection or the infusion solution for drip infusion include distilled water for injection, physiological saline, glucose solution and isotonic solution and the like (e.g., solutions in which sodium chloride, potassium chloride, glycerin, mannitol, sorbitol, boric acid, borax, propylene glycol or the like is dissolved.), which may contain a pharmaceutically acceptable carrier, if necessary. Herein, examples of the pharmaceutically acceptable carriers include a stabilizer, solubilizer suspending agent, emulsifier, soothing agent, buffering agent, preservative, antiseptic agent, pH adjuster, antioxidant and the like. As a stabilizer, for example, various amino acids, albumin, globulin, gelatin, mannitol, glucose, dextran, ethylene glycol, propylene glycol, polyethylene glycol, ascorbic acid, sodium bisulfite, sodium thiosulfate, sodium edetate, sodium citrate, dibutylhydroxytoluene or the like can be used. As a solubilizer, for example, alcohol (e.g., ethanol etc.), polyalcohol (e.g., propylene glycol and polyethylene glycol, etc.), nonionic surfactant (e.g., Polysorbate 20 (registered trademark), Polysorbate 80 (registered trademark) and HCO-50, etc.) or the like can be used. As a suspending agent, for example, glyceryl monostearate, aluminum monostearate, methyl cellulose, carboxymethyl cellulose, hydroxymethyl cellulose, sodium lauryl sulfate or the like can be used. As an emulsifier, for example, gum arabic, sodium alginate, tragacanth or the like can be used. As a soothing agent, for example, benzyl alcohol, chlorobutanol, sorbitol or the like can be used. As a buffering agent, for example, phosphate buffer, acetate buffer, borate buffer, carbonate buffer, citrate buffer, Tris buffer, glutamic acid buffer, epsilon aminocaproic acid buffer or the like can be used. As a preservative, for example, methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edeate, boric acid, borax or the like can be used. As an antiseptic agent, for example, benzalkonium chloride, parahydroxybenzoic acid, chlorobutanol or the like can be used. As a pH adjuster, for example, hydrochloric acid, sodium hydroxide, phosphoric acid, acetic acid or the like can be used. As an antioxidant, for example, (1) aqueous antioxidants such as ascorbic acid, cysteine hydrochloride, sodium bisulfate, sodium metabisulfite and sodium sulfite, (2) oil-soluble antioxidants such as ascorbyl palmitate, butylated hydroxy anisole, butylated hydroxy toluene, lecithin, propyl gallate and a-tocopherol, and (3) metal chelating agents such as citric acid, ethylenediaminetetraacetic acid, sorbitol, tartaric acid and phosphoric acid can be used.

The injection or infusion solution for drip infusion can be produced by performing sterilization in the final process, or sterilization by aseptic manipulation, for example, sterilization by filtration with a filter or the like and subsequently filling it to an aseptic container.

The contents of all patents and non-patent literature or references explicitly cited in the present specification may all be cited herein as part of the present specification.

The present invention will now be described in more detail by the following examples, but the scope of the present invention is not limited thereto. A person skilled in the art can make various changes and modifications, based on the description of the present invention, and such changes and modifications are also included in the present invention.

### [Examples]

### Example 1: Quantitative analysis of colored antibodies by reversed-phase HPLC

In order to quantitatively evaluate the amount of colored bodies of anti-PD-1/CD3 bispecific antibody, an evaluation system using reversed-phase HPLC was constructed. Tables 1 and 2 below show the equipment used on that and conditions set.

**[Table 1]**

| HPLC System Components | Details |
|---|---|
| Controller | CBM-20A (Shimadzu Corp.) |
| Pump | LC-20AD (Shimadzu Corp.) |
| Autosampler | SIL-20AC (Shimadzu Corp.) |
| Column Oven | CTO-20AC (Shimadzu Corp.) |
| UV Detector | SPD-20A (Shimadzu Corp.) |
| Fluorescence Detector | RF-20AXS (Shimadzu Corp.) |

**[Table 2]**

| Items | Details |
|---|---|
| Column | Aeris WIDEPORE XB-C8 150 ×2.1 mmI.D., 3.6µm |
| Sample | 5µg of antibody |
| Column Temp. | 75°C |
| Flow rate | 0.33 mL/min |
| Mobile phase A | 0.1% TFA in water: isopropyl alcohol = 98 : 22 |
| Mobile phase B | 0.1% TFA in isopropyl alcohol: acetonitrile: water = 7 : 2. : 1 |
| Gradient | 10% B in 0 - 1.5 min |
| | 10 - 25% B in 1.5 - 3.0 min |
| | 25 - 40% B in 3.0 - 18.75 min |
| | 40 - 40% B in 18.75 - 33.75 min |
| | 100% B in 33.75 - 44.0 mm |
| | 10% B in 44.0 - 55.0 min |
| UV detection | Wavelength: 280nm, Cell Temp.: 40°C, Range: 1.0AU/V, Output range: 1.0000 |
| Fluorescence detection | Excitation wavelength: 380 nm, Fluorescence wavelength: 560 nm Cell temp.: 30°C, Gain × 1 Sensitivity: High |

The analysis was started by loading a solution of 5 µg equal volume of anti-PD-1/CD3 bispecific antibody A into the column, sufficiently stabilized at the predetermined column temperature and with the mobile phase A. From the obtained chromatogram, a predefined target peak was cut vertically at the position where the detection signal was the lowest compared to the adjacent peaks before and after the same peak, and the UV detection peak area at 280 nm wavelength (UV280) and the fluorescence intensity detection peak area (Em 560(ex380)) were obtained, and then the Em/UV*100 value was calculated and used as an index for color quantification. Note that because the fluorescence detector is connected after the UV detector in the system, the fluorescence detection peak appears later.

Using this method, the Em/UV^{∗}100 values were compared with those of the reference standard solutions in item 2.65 "color comparison test" of the general test methods of the Japanese Pharmacopoeia, 17th Edition. The anti-PD-1/CD3 bispecific antibody purified under a condition set without attention to the removal of colorants was fractionated by strong cation-exchange chromatography with a salt concentration gradient, and the purified fraction obtained was concentrated to 100 mg/mL. By mixing the highly colored fraction and low colored fraction thereof, samples with colorations corresponding to the reference solutions BY3.5, BY4, BY4.5, BY5 and BY6 were prepared based on visual inspection, then which were analyzed by HPLC. Comparing the chromatograms corresponding to BY3.5 (Figure 1) and BY6.0 (Figure 2), it is clear that the Em/UV^{∗}100 value in the sample corresponding to BY3.5 is larger. By comparing the HPLC analysis sample with the reference standard solution, it was confirmed that the Em/UV^{∗}100 value should be about 6 or less when the clarity of a protein subject to the method of the present invention is made to be lighter than that of BY5 in the reference standard solution (Figure 3).

### Example 2: Antibody purification by anion-exchange chromatography in the Bind-elute mode (1)

In order to find a separation condition to reduce the ratio of colorant of the anti-PD-1/CD3 bispecific antibody A pertaining to the present invention, the pH step gradient anion-exchange chromatography in the Bind-elute mode was performed (Figure 4). Herein, a separation column packed with Toyopearl NH2-750F on Tricorn column (GE Healthcare) by flow packing was used. A solution containing the anti-PD-1/CD3 bispecific antibody A purified by Protein A affinity chromatography from clarified CHO cell culture medium was prepared to be at pH 10.2 after low pH treatment for virus inactivation, and used as a sample before anion-exchange chromatography purification. Table 3 shows the other equipment used herein and conditions set, respectively.

**[Table 3]**

| Purification conditions | Details |
|---|---|
| Column | Toyopearl NH2-750F (100 x 5mm I.D. CV:2mL) |
| System | AKTA avant |
| Sample | 20 mg-IgG/mL-resin, pH 10.2 |
| Mobile phase A | Commercial buffer A, pH 10.2 (Thermo Fisher) |
| Mobile phase B | Commercial buffer B, pH 5.6 (Thermo Fisher) |
| Flow rate | 0.5 mL/min |
| Gradient | 25, 26, 27, 28, 29, 30, 31 % B in 4CV |

After sufficiently equilibrating the column with commercial buffer A (Thermo Scientific (registered trademark) CX-1 pH Gradient Buffer B (pH 10.2) from Thermo Fisher) to be used as the mobile phase A, 4.3 mL-weighed antibody solution at 9.3 mg/mL was fully loaded so that the loading volume thereof is made to be at 20 mg-IgG/mL-resin. Then, when the ratio of commercial buffer B (Thermo Scientific (registered trademark) CX-1 pH Gradient Buffer A (pH 5.6) from Thermo Fisher (%B in Figure 4) corresponding to the mobile phase B was increased step by step from 25%, a peak rose sharply from around 26 (%B), and a main peak of the antibody appeared at around 20 mL of column volume. The Em/UV^{∗}100 values of fractions near the peak were 1.4, 1.4, 5.1 and 7.6, respectively (corresponding to the arrows 1 to 4 in Figure 4, respectively), starting from the weakly retained fraction. Since the Em/UV^{∗}100 value of a pre-purified sample of the antibody was 8.6, it was confirmed that the colorant has been highly separated by the method of the present example.

### Example 3: Antibody purification by anion-exchange chromatography in the Flow-through mode

Furthermore, in order to find another separation condition to reduce the ratio of colorant of the anti-PD-1/CD3 bispecific antibody A, the anion-exchange chromatography in the Flow-through mode was performed using the same separation column system as in Example 1 (Figure 5). Similarly, a solution containing the anti-PD-1/CD3 bispecific antibody A purified by Protein A affinity chromatography from clarified CHO cell culture medium was prepared to be at pH 8.0 after low pH treatment for virus inactivation, and used as a sample before anion-exchange chromatography purification. Table 4 shows the other equipment used herein and conditions set, respectively.

**[Table 4]**

| Purification conditions | Details |
|---|---|
| Column | Toyopearl NH2-750F (100 x 5mm I.D. CV:2mL) |
| System | AKTA avant |
| Sample | 20 mg-IgG/mL-resin, pH 8.0 |
| Mobile phase A | HEPES buffer, pH 8.0 |
| Mobile phase B | Commercial buffer B, pH 5.6 (Thermo Fisher) |
| Flow rate | 0.5 mL/min |
| Gradient | 0 % B in 10CV, 0→100 % B in 10 CV, 100 % B in 10 CV |

After sufficiently equilibrating the column with HEPES buffer (pH 8.0) to be used as the mobile phase A, 3.9 mL-weighed antibody solution at 10.3 mg/mL was fully loaded so that the loading volume thereof is made to be at 20 mg-IgG/mL-resin. Immediately after passing the solution, a flow-through peak containing the antibody appeared. Then, when the pH was continuously lowered with the mobile phase B (commercial buffer B (Thermo Scientific (registered trademark) CX-1 pH Gradient Buffer A (pH 5.6) from ThermoFisher), an elution peak presumed to be an impurity which had been adsorbed appeared. While the Em/UV* 100 value of the fraction containing the Flow-through peak was 4.0, since the Em/UV* 100 values of a pre-purified antibody solution and predefined fraction in the elution peak were 9.1 and 22.2, respectively, it was confirmed that the colorant has been highly separated in the method of the present example, as well.

### Example 4: Antibody purification by anion-exchange chromatography in the Bind-elute mode (2)

In order to find another separation condition to reduce the ratio of colorant in the anti-PD-1/CD3 bispecific antibody, the anion-exchange chromatography in the Bind-elute mode was performed using a separation column packed with Capto adhere on Tricorn column (GE Healthcare) by flow packing (Figure 6). A solution containing the anti-PD-1/CD3 bispecific antibody A purified by Protein A affinity chromatography from clarified CHO cell culture medium was prepared to be at pH 10.2 after low pH treatment for virus inactivation, and used as a sample before anion-exchange chromatography purification. Table 5 shows the other equipment used herein and conditions set, respectively.

**[Table 5]**

| Purification conditions | Details |
|---|---|
| Column | Capto adhere (100 x 5mm I.D, CV:2mL) |
| System | AKTA avant |
| Sample | 20 mg-IgG/mL-resin, pH 10.2 |
| Mobile phase A | Commercial buffer A, pH 10.2 (Thermo Fisher) |
| Mobile phase B | Commercial buffer B, pH 5.6 (Thermo Fisher) |
| Flow rate | 0.5 mL/min |
| Gradient | 25 %B in 4CV, 25→100 %B in 75CV, 100 %B in 8CV |

After sufficiently equilibrating the column with commercial buffer A (pH 10.2) to be used as the mobile phase A, 3.8 mL-weighed antibody solution at 10.6 mg/mL was fully loaded so that a loading volume is made to be at 20 mg-IgG/mL-resin. Then, the ratio of commercial buffer B corresponding to the mobile phase B (%B in Figure 6) was set to 25 (%B) and held for 4 column volumes, and then when the ratio of the mobile phase B was continuously increased from 25 (%B), a peak of the antibody appeared at or after 40 mL of column volume. The Em/UV*100 values of fractions near the peak were 1.7, 17, 6.5, 10.8 and 14.4, respectively (corresponding to the arrows 1 to 5 in Figure 6, respectively), starting from the weakly retained fraction. Since the Em/UV*100 value of a pre-purified antibody sample was 9.0, it was confirmed that the colorant has been highly separated by the method of the present example, as well.

### Example 5: Antibody purification by anion-exchange chromatography in the Bind-elute mode (3)

Subsequently, the anion-exchange chromatography in the Bind-elute mode was performed using a separation column packed with Cellufine MAX IB on Tricorn column (GE Healthcare) by flow packing (Figure 7). The solution containing the anti-PD-1/CD3 bispecific antibody A purified by Protein A affinity chromatography from clarified CHO cell culture medium was prepared to be at pH 10.2 after low pH treatment for virus inactivation, and used as a sample before anion-exchange chromatography purification. Table 6 shows the other equipment used herein and conditions set, respectively.

**[Table 6]**

| Purification conditions | Details |
|---|---|
| Column | Cellufine MAX IB (100 x 5mm I.D. CV: 2mL) |
| System | AKTA avant |
| Sample | 20 mg-IgG/mL-resin, pH 10.2 |
| Mobile phase A | Commercial buffer A. pH 10.2 (Thermo Fisher) |
| Mobile phase B | Commercial buffer B, pH 5.6 (Thermo Fisher) |
| Flow rate | 0.5 mL/min |
| Gradient | 25 %B in 4CV, 25->100 %B in 75CV, 100 %B in 8CV |

After sufficiently equilibrating the column with commercial buffer A (pH 10.2) to be used as mobile phase A, 3.8 mL-weighed antibody solution at 10.6 mg/mL was fully loaded so that a loading volume is made to be at 20 mg-IgG/mL-resin. Then, the ratio of commercial buffer B corresponding to the mobile phase B (%B in Figure 7) was set to 25 (%B) and held for 4 column volumes, and then when the ratio of the mobile phase B was continuously increased, a peak of the antibody appeared at or after about 30 mL of column volume. The Em/UV^{∗}100 values of fractions near the peak were 1.6, 17, 2.1, 3.3 and 8.0, respectively (corresponding to the arrows 1 to 5 in Figure 7, respectively), starting from the weakly retained fraction. Since the Em/UV* 100 value of the pre-purified antibody sample was 9.0, it was confirmed that the colorant has been highly separated by the method of the present example, as well.

### Example 6: Anion-exchange chromatographic purification of BSA

In order to verify whether it is possible to separate colorants from proteins other than antibodies, as with the case of antibodies, the anion-exchange chromatography in the Bind-elute mode was performed on BSA (bovine serum albumin; low salt concentration, Fujifilm Wako Pure Chemicals) containing colorants (Fig. 8). Herein, a separation column packed with Capto Q on Tricorn column (GE Healthcare) was used, and Table 7 shows the other equipment used herein and conditions set, respectively.

**[Table 7]**

| Purification conditions | Details |
|---|---|
| Column | Capto Q (150 x 10mm I.D. CV: 11.8 mL) |
| System | AKTA avant |
| Sample | 40 mg-BSA/mL-resni, pH 7.0 |
| Mobile phase A | Citric acid-phosphate buffer pH 7.0 |
| Mobile phase B | Citric acid-phosphate buffer pH 2.6 |
| Flow rate | 2.9 mL/min |
| Gradient | 70 %B in 4CV, 70->100 %B in 30CV, 100 %B in 5CV |

After sufficiently equilibrating the column with citric acid-phosphate buffer (pH 7.0) corresponding to the mobile phase A, 157 mL-weighed BSA solution at 3.0 mg/mL was fully loaded so that a loading volume is made to be at 40 mg-BSA/mL-resin. Then, the ratio (%B) of the mobile phase B was set to 70 (%B) and held for 4 column volumes, and then when the ratio of the mobile phase B was continuously increased, a peak of BSA appeared at or after about 180 mL of column volume. The Em/UV* 100 values of fractions near the peak were 7.7, 17, 10.3 and 13.8, respectively (corresponding to the arrows 1 to 3 in Figure 8, respectively), starting from the weakly retained fraction. Since the Em/UV^{∗}100 value of a pre-purified BSA sample was 10.7, it was confirmed that for not only antibodies but also other proteins, colorants thereof have been highly separated by the method of the present example.

### [Industrial Applicability]

The present separation method can be used to remove the color of colorless protein solutions, especially antibody drugs which require strict quality control.

## Claims

1. A method for preparing a solution containing non-colored proteins comprising a process for separating non-colored proteins from a solution containing non-colored proteins and colored proteins, the separation process comprising
(i) a step for loading the solution onto an ion-exchange carrier column, and using an equilibration buffer at a predetermined initial pH, making the non-colored proteins and colored proteins bind to ion-exchange carriers,
(ii) a step for flowing an elution buffer, forming a pH gradient varying from the initial pH to a terminal pH, through the ion-exchange carrier column at a predetermined flow rate, under a condition predetermined to separate the non-colored proteins and colored proteins, and
(iii) a step for collecting a predetermined fraction containing the non-colored proteins eluted by the preceding step (ii).

2. A method for preparing a solution containing non-colored proteins comprising a process for separating non-colored proteins from a solution containing non-colored proteins and colored proteins, the separation process comprising
(i) a step for loading the solution onto an ion-exchange carrier column,
(ii) a step for flowing an equilibration buffer prepared so that the colored proteins in the solution bind to ion-exchange carriers and the non-colored proteins do not bind, through the ion-exchange carrier column at a predetermined flow rate, and
(iii) a step for collecting a fraction containing the non-colored proteins flowed through in the preceding step (ii) as it is.

3. The preparation method according to claim 1 or 2, wherein the color concentration of the solution containing non-colored proteins and colored proteins or concentrated solution thereof is (1) in the visual comparison test with the reference standard solution BY5 of the color comparison test described in item 2.65 of the general tests of the Japanese Pharmacopoeia, 17th Edition, more than that of BY5, or (2) the value calculated by multiplying the ratio of the peak area of fluorescence intensity (provided that the excitation wavelength is 380 nm and the detection wavelength is 560 nm) to the peak area of UV absorbance at a wavelength of 280 nm, calculated based on the UV absorption measurement and fluorescence spectrum measurement of the solution or concentrated solution thereof, by 100 (Em/UV* 100 value) is higher than about 6.3.

4. The preparation method according to any one of claims 1 to 3, wherein the ion-exchange carrier is an anion-exchange carrier.

5. The preparation method according to claim 4, wherein the anion-exchange carrier is a weak anion-exchange carrier or multi-mode anion-exchange carrier.

6. The preparation method according to claim 5, wherein the weak anion-exchange carrier is selected from the group consisting of Fractogel (registered trademark) EMD DEAE, Fractogel (registered trademark) EMD DMAE, Capto (registered trademark) DEAE, DEAE Ceramic HyperD (registered trademark) F, Toyopearl (registered trademark) NH2-750F, TOYOPEARL (registered trademark) DEAE-650C, TOYOPEARL (registered trademark) DEAE-650M, TOYOPEARL (registered trademark) DEAE-650S, Cellufine (registered trademark) A-200, Cellufine (registered trademark) A-500, Cellufine (registered trademark) A-800 and Cellufine (registered trademark) MAX DEAE.

7. The preparation method according to claim 5, wherein the multi-mode anion-exchange carrier is selected from the group consisting of Toyopearl (registered trademark) NH2-750F, Capto (registered trademark) Adhere, Capto (registered trademark) Adhere ImpRes, Capto (registered trademark) MMC, Capto (registered trademark) core 700 and Cellufine (registered trademark) IB.

8. The preparation method according to any one of claims 1 to 7, wherein the loading amount of the non-colored proteins and colored proteins which are loaded into the ion-exchange carrier column is from about 2 to about 200 mg/mL.

9. The preparation method according to any one of claims 1 and 3 to 8, wherein the range of the pH gradient from the initial pH to the terminal pH is set so that the respective isoelectric points of the colored protein and/or the non-colored protein is/are interposed between the same range.

10. The preparation method according to any one of claims 1 and 3 to 9, wherein the pH gradient descends from the initial pH to the terminal pH.

11. The preparation method according to any one of claims 1 and 3 to 10, wherein the initial pH is an arbitrary pH within the range of from about 11.0 to about 7.0.

12. The preparation method according to any one of claim 1 and 3 to 11, wherein the terminal pH is an arbitrary pH within the range of from about 4.0 to about 8.2 (provided that the initial pH is at least 1.0 higher than the terminal pH).

13. The preparation method according to any one of claims 1 and 3 to 12, wherein the pH gradient is a linear gradient or step-wise gradient.

14. The preparation method according to any one of claims 1 and 3 to 13, wherein the elution buffer consists of a combination of the buffer at the initial pH and the buffer at the terminal pH at an arbitrary ratio thereof.

15. The preparation method according to claim 14, wherein the buffer at the initial pH and/or the buffer at the terminal pH consist(s) of a combination of a plurality of good buffers selected from the group consisting of MES, Bis-Tris, ADA, PIPES, ACES, MOPSO, BES, MOPS, TES, HEPES, TAPSO, POPSO, HEPSO, EPPS, Tricine, Bicine, TAPS, CHES, CAPSO and CAPS, respectively.

16. The preparation method according to claim 14, wherein the buffer at the initial pH is Thermo Scientific (registered trademark) CX-1 pH Gradient Buffer B (pH 10.2) and the buffer at the terminal pH is Thermo Scientific (registered trademark) CX-1 pH Gradient Buffer A (pH 5.6).

17. The preparation method according to any one of claims 14 to 16, wherein the pH gradient with the elution buffer is performed by continuously or step-by-step changing the ratio of the combination of the buffer at the initial pH and the buffer at the terminal pH.

18. The preparation method according to any one of claims 14 to 17, wherein the pH gradient is performed (a) by increasing the percentage (%) of the buffer at the terminal pH in the elution buffer step-by-step by an arbitrary percentage in the range of from about 1 to about 2 %, at an interval corresponding to an arbitrary column volume in the range of from about 4 to about 6 CV, over at least the range of from about 50 to 100%, or (b) by decreasing the pH of the elution buffer step-by-step by an arbitrary pH in the range of from about 0.6 to about 1.0, within a range corresponding to an arbitrary column volume of from about 30 to about 40 CV.

19. The preparation method according to any one of claims 1 to 18, wherein the respective linear flow rates of the elution buffer described in claim 1 and the equilibration buffer described in claim 2 are arbitrary linear flow rates in the range of from about 100 to about 800 cm/h.

20. The preparation method according to any one of claims 1 and 3 to 19, wherein the electrical conductivity of the equilibration buffer and/or elution buffer described in claim 1 is an arbitrary electrical conductivity in the range of from about 1 to about 20 mS/cm.

21. The preparation method according to any one of claims 2 to 8 and 19, wherein the electrical conductivity of the equilibration buffer described in claim 2 is an arbitrary electrical conductivity in the range of from about 1 to about 20 mS/cm.

22. The preparation method according to any one of claims 2 to 8, 19 and 21, wherein the pH of the equilibration buffer described in claim 2 is an arbitrary pH in the range of from about 9.2 to about 7.4.

23. The preparation method according to any one of claims 1 to 22, which includes a step for washing the ion-exchange carrier with a washing buffer, as necessary, in each step included in the separation process described in any one of claims 1 to 22.

24. The preparation method according to any one of claims 1 to 23, wherein the color concentration of the collected or recovered fraction of the non-colored proteins or concentrated solution thereof is substantially the same as or lighter than that of BY5 in the visual comparison test with the reference standard solution BY5 in the "color comparison test" of the Japanese Pharmacopoeia.

25. The preparation method according to any one of claims 1 to 23, wherein the color concentration of the collected or recovered fraction of the non-colored proteins or concentrated solution thereof is substantially the same as or lighter than that of BY6 in the visual comparison test with the reference standard solution BY6 in the "color comparison test" of the Japanese Pharmacopoeia.

26. The preparation method according to any one of claims 1 to 23, wherein the color concentration of the collected or recovered fraction of the non-colored proteins or concentrated solution thereof is substantially the same as that of BY7 in the visual comparison test with the reference standard solution BY7 in the "color comparison test" of the Japanese Pharmacopoeia.

27. The preparation method according to any one of claims 1 to 23, wherein the Em/UV*100 value of the collected or recovered fraction of the non-colored proteins or concentrated solution thereof is about 6.3 or less.

28. The preparation method according to any one of claims 1 to 23, wherein the Em/UV*100 value of the collected or recovered fraction of the non-colored proteins or concentrated solution thereof is about 4.1 or less.

29. The preparation method according to any one of claims 1 to 28, wherein the protein concentration of the collected or recovered fraction of the non-colored proteins is an arbitrary concentration in the range of from about 20 to about 300 mg/mL.

30. The preparation method according to any one of claims 1 to 29, wherein the protein is an antibody or antibody fragment thereof.

31. The preparation method according to claim 30, wherein the antibody is a bispecific antibody.

32. The preparation method according to claim 31, wherein the bispecific antibody is a bispecific antibody capable of specifically binding to PD-1 and CD3, respectively.

33. The preparation method according to claim 32, wherein the bispecific antibody capable of specifically binding to PD-1 and CD3, respectively, comprises a heavy chain and light chain constituting an antigen-binding site specifically binding to PD-1, and a heavy chain and light chain constituting an antigen-binding site specifically binding to CD3, and wherein (a) the heavy chain of the heavy chain and light chain constituting an antigen-binding site specifically binding to PD-1 comprises the amino acid sequence set forth in any one selected from SEQ ID No. 1, SEQ ID No. 2, SEQ ID No. 3, SEQ ID No. 4 and SEQ ID No. 5, (b) the heavy chain of the heavy chain and light chain constituting an antigen-binding site specifically binding to CD3 comprises the amino acid sequence set forth in SEQ ID No. 6, and (c) both of the light chain of the heavy chain and light chain constituting an antigen-binding site specifically binding to PD-1 and the light chain of the heavy chain and light chain constituting an antigen-binding site specifically binding to CD3 comprise the amino acid sequence set forth in SEQ ID No. 7.

34. The preparation method according to claim 33, wherein the heavy chain of the heavy chain and light chain constituting an antigen-binding site specifically binding to PD-1 comprises the amino acid sequence set forth in SEQ ID No. 5.

35. The preparation method according to claim 33 or 34, wherein a heavy chain constant region in each heavy chain of the heavy chain and light chain constituting an antigen-binding site specifically binding to PD-1 described in claim 33 or 34 is substituted with a heavy chain constant region comprising the amino acid sequence set forth in any one selected from SEQ ID No. 8, SEQ ID No. 9, SEQ ID No. 10, SEQ ID No. 11, SEQ ID No. 12 and SEQ ID No. 13, and a heavy chain constant region in each heavy chain of the heavy chain and light chain constituting an antigen-binding site specifically binding to CD3 described in the same, is substituted with a heavy chain constant region comprising the amino acid sequence set forth in any one selected from SEQ ID No. 14, SEQ ID No. 15, SEQ ID No. 16, SEQ ID No. 17, SEQ ID No. 18 and SEQ ID No. 19.

36. A method for preparing a solution containing non-colored proteins comprising a process for separating non-colored proteins from a solution containing non-colored proteins and colored proteins, (1) wherein the separation process comprises
(i) a step for loading the solution onto an anion-exchange carrier column, and using an equilibration buffer at a predetermined initial pH, making the non-colored proteins and colored proteins bind to anion-exchange carriers,
(ii) a step for flowing an elution buffer, forming a pH gradient which descends from the initial pH to a terminal pH, through the anion-exchange carrier column at a predetermined flow rate, under a condition predetermined to separate the non-colored proteins and colored proteins; and
(iii) a step for collecting a predetermined fraction containing the non-colored proteins eluted by the preceding step (ii),
(2) wherein the protein is a bispecific antibody specifically binding to PD-1 and CD3, respectively, comprising a heavy chain and light chain constituting an antigen-binding site specifically binding to PD-1 and a heavy chain and light chain constituting an antigen-binding site specifically binding to CD3, and wherein (a) the heavy chain of the heavy chain and light chain constituting an antigen-binding site specifically binding to PD-1 comprises the amino acid sequence set forth in any one selected from SEQ ID No. 1, SEQ ID No. 2, SEQ ID No. 3, SEQ ID No. 4 and SEQ ID No. 5, (b) the heavy chain of the heavy chain and light chain constituting an antigen-binding site specifically binding to CD3 comprises the amino acid sequence set forth in SEQ ID No. 6, and (c) both of the light chain of the heavy chain and light chain constituting an antigen-binding site specifically binding to PD-1 and the light chain of the heavy chain and light chain constituting an antigen-binding site specifically binding to CD3 comprise the amino acid sequence set forth in SEQ ID No. 7,
(3) wherein the anion-exchange carrier is Toyopearl (registered trademark) NH2-750F,
(4) wherein the initial pH of the equilibration buffer and elution buffer is an arbitrary pH in the range of from about 10.6 to about 8.4, and the terminal pH of the elution buffer is an arbitrary pH in the range of from about 7.0 to about 4.0 (provided that the initial pH is at least 2.0 higher than the terminal pH),
(5) wherein the pH gradient is performed (a) by increasing the percentage (%) of the buffer B in the elution buffer step-by-step by an arbitrary percentage in the range of from about 1 to about 2 %, at an interval corresponding to an arbitrary column volume in the range of from about 4 to about 6 CV, over at least the range of from about 50 to 100%, or (b) by decreasing the pH of the elution buffer step-by-step by an arbitrary pH in the range of from about 0.6 to about 1.0, within a range corresponding to an arbitrary column volume of from about 30 to about 40 CV,
(6) wherein the linear flow rate of the elution buffer is from about 100 to about 800 cm/h, and
(7) wherein the color concentration of the collected solution containing the non-colored proteins is (i) substantially the same as or lighter than that of BY5 in the visual comparison test with the reference standard solution BY5 of the color comparison test described in item 2.65 of the general tests of the Japanese Pharmacopoeia, 17th Edition or (ii) the Em/UV* 100 value of the collected solution containing the non-colored proteins is about 6.3 or less.

37. A method for preparing a solution containing non-colored proteins comprising a process for separating non-colored proteins from a solution containing non-colored proteins and colored proteins, (1) wherein the separation process comprises
(i) a step for loading the solution onto an anion-exchange carrier column,
(ii) a step for flowing an equilibration buffer prepared so that the colored proteins in the solution bind to anion-exchange carriers and the non-colored proteins do not, through the anion-exchange carrier column at a predetermined flow rate, and
(iii) a step for collecting the fraction containing the non-colored proteins flowed through in the preceding step (ii) as it is,
(2) wherein the protein is a bispecific antibody specifically binding to PD-1 and CD3, respectively, comprising a heavy chain and light chain constituting an antigen-binding site specifically binding to PD-1 and a heavy chain and light chain constituting an antigen-binding site specifically binding to CD3, and wherein (a) the heavy chain of the heavy chain and light chain constituting an antigen-binding site specifically binding to PD-1 comprises the amino acid sequence set forth in any one selected from SEQ ID No. 1, SEQ ID No. 2, SEQ ID No. 3, SEQ ID No. 4 and SEQ ID No. 5, (b) the heavy chain of the heavy chain and light chain constituting an antigen-binding site specifically binding to CD3 comprises the amino acid sequence set forth in SEQ ID No. 6, and (c) both of the light chain of the heavy chain and light chain constituting an antigen-binding site specifically binding to PD-1 and the light chain of the heavy chain and light chain constituting an antigen-binding site specifically binding to CD3 comprise the amino acid sequence set forth in SEQ ID No. 7,
(3) wherein the anion-exchange carrier is Toyopearl (registered trademark) NH2-750F,
(4) wherein the pH of the equilibration buffer is an arbitrary pH in the range of from about 7.8 to about 8.2, and the electrical conductivity thereof is an arbitrary electrical conductivity from about 5 to about 7 mS/cm,
(5) wherein the linear flow rate of the equilibration buffer is from about 100 to about 400 cm/h, and
(6) wherein the color concentration of the collected solution containing the non-colored proteins is (i) substantially the same as or lighter than that of BY5 in the visual comparison test with the reference standard solution BY5 of the color comparison test described in item 2.65 of the general tests of the Japanese Pharmacopoeia, 17th Edition, or (ii) the Em/LTV* 100 value of the collected solution containing the non-colored protein is about 6.3 or less.

38. A colorless or slightly colored solution comprising a bispecific antibody specifically binding to PD-1 and CD3, respectively, at a concentration of from about 100 to about 300 mg/mL, wherein the bispecific antibody specifically binding to PD-1 and CD3, respectively, comprises the heavy chain and light chain constituting an antigen-binding site specifically binding to PD-1 and the heavy chain and light chain constituting an antigen-binding site specifically binding to CD3, and wherein (a) the heavy chain of the heavy chain and light chain constituting an antigen-binding site specifically binding to PD-1 comprises the amino acid sequence set forth in any one selected from SEQ ID No. 1, SEQ ID No. 2, SEQ ID No. 3, SEQ ID No. 4 and SEQ ID No. 5, (b) the heavy chain of the heavy chain and light chain constituting an antigen-binding site specifically binding to CD3 comprises the amino acid sequence set forth in SEQ ID No. 6, and (c) both of the light chain of the heavy chain and light chain constituting an antigen-binding site specifically binding to PD-1 and the light chain of the heavy chain and light chain constituting an antigen-binding site specifically binding to CD3 comprise the amino acid sequence set forth in SEQ ID No. 7.

39. The solution according to claim 38, wherein the color concentration of the solution is (i) substantially the same as or lighter than that of BY5 in the visual comparison test with the reference standard solution BY5 of the color comparison test described in item 2.65 of the general tests of the Japanese Pharmacopoeia, 17th Edition, or (ii) the Em/UV*100 value of the collected solution containing the non-colored proteins is about 6.3 or less.

40. The solution according to claim 38 or 39, except for solutions which are colorless or slightly colored without any separation process to reduce or remove the coloration.

41. A colorless or slightly colored solution comprising the bispecific antibody specifically binding to PD-1 and CD3, respectively, described in any one of claims 33 to 35, and prepared by the method described in any one of claims 1 to 37.

42. A pharmaceutical composition comprising the solution described in any one of claims 38 to 41.

43. Use of the pharmaceutical composition described in claim 42, in preventing, suppressing the symptom progression of, suppressing the recurrence of, and/or treating autoimmune disease or graft-versus-host disease or hematological cancer.
